(19) 

Europäisches
Patentamt
European
Patent Office
Office européen
des brevets

(11) **EP 4 190 816 A1**

(12) **EUROPEAN PATENT APPLICATION**
published in accordance with Art. 153(4) EPC

(43) Date of publication:
**07.06.2023 Bulletin 2023/23**

(21) Application number: **22787606.7**

(22) Date of filing: **14.04.2022**

(51) International Patent Classification (IPC):
**C07K 16/28** (2006.01) **C12N 15/13** (2006.01)
**C07K 19/00** (2006.01)

(52) Cooperative Patent Classification (CPC):
**A61K 31/69; A61K 31/7068; A61K 39/395;
A61K 45/06; A61P 35/00; A61P 35/02;
C07K 16/00; C07K 16/28; C07K 16/46; C07K 19/00**

(86) International application number:
**PCT/CN2022/086890**

(87) International publication number:
**WO 2022/218384 (20.10.2022 Gazette 2022/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.04.2021 CN 202110404033**

(71) Applicants:
• **Akeso Biopharma, Inc.**
**Zhongshan, Guangdong 528437 (CN)**
• **Akeso Pharmaceuticals, Inc.**
**Guangdong 510799 (CN)**

(72) Inventors:
• **LI, Baiyong**
**Zhongshan Guangdong 528437 (CN)**
• **XIA, Yu**
**Zhongshan Guangdong 528437 (CN)**
• **WANG, Zhongmin Maxwell**
**Zhongshan Guangdong 528437 (CN)**

(74) Representative: **Cooley (UK) LLP**
**22 Bishopsgate**
**London EC2N 4BQ (GB)**

Remarks:
The complete document including Reference
Table(s) and the Sequence Listing(s) can be
downloaded from the EPO website

(54) **ANTI-CD47 MONOCLONAL ANTIBODY AND USE THEREOF**

(57) An anti-CD47 monoclonal antibody and the use thereof in combination with a monoclonal antibody, a bispecific antibody and/or a tumor therapeutic agent, wherein the anti-CD47 monoclonal antibody is secreted by a hybridoma cell line with a deposit number of CCTCC NO: C2018135.

EP 4 190 816 A1

**Description**

**TECHNICAL FIELD**

The present invention relates to the fields of autoimmune diseases treatment and molecular immunology, and particularly to an anti-CD47 antibody, a pharmaceutical composition comprising the same, and use thereof. More particularly, the present invention relates to an anti-CD47 monoclonal antibody.

**BACKGROUND**

CD47 is also referred to as integrin associated protein (IAP). CD47 is a five-span transmembrane protein with a molecular weight of about 50 kDa and belongs to the immunoglobulin superfamily. Its extracellular N terminus is an IgV domain and is connected to $\alpha v\beta 3$ (CD51/CD61) and $\alpha IIb\beta 3$ (CD41/CD61) integrins. CD47 is involved in a variety of physiological functions, such as cell transfer, T cell and dendritic cell (DC) activation, and axonal development.

CD47 is expressed on all types of cells including erythrocytes, and is highly expressed on tumor cells. CD47 has two ligands, namely signal regulatory protein-a (SIRP$\alpha$) and thrombospondin-1 (TSP1). SIRP$\alpha$, a receptor transmembrane glycoprotein comprises an immunoglobulin domain, belongs to the SIRP family, and is mainly expressed on macrophages and nerve cells. In the CD47-SIRP$\alpha$ pathway, CD47 protein binds to SIRP$\alpha$ and phosphorylates its immunoreceptor tyrosine-based inhibitory motif (ITIM), and intracellularly recruits SHP-1 protein to produce a series of cascade reactions to inhibit macrophage phagocytosis (Matozaki T, Murata Y, Okazawa H, et al., Functions and molecular mechanisms of the CD47-SIRP$\alpha$ signaling pathway. Trends in cell biology, 2009, 19(2): 72-80.). However, normal red blood cells are not phagocytosed due to the inhibitory signal generated by the binding of CD47 on the surface of the cell membrane to SIPR$\alpha$ of macrophages. (Oldenborg P A, Zheleznyak A, Fang Y F, et al., Role of CD47 as a marker of self on red blood cells. Science, 2000, 288(5473): 2051-2054.). TSP1, a homotrimer composed of 3 peptide chains, is involved in cell proliferation, apoptosis, adhesion, migration, angiogenesis and other processes through interaction with other cell surface receptors, matrix components and growth factors (Jiang P, Lagenaur CF, Narayanan V. Integrin-associated Protein Is a Ligand for the P84 Neural Adhesion Molecule. Journal of Biological Chemistry 1999. 274:559-62). Macrophages are derived from monocytes, which in turn are derived from precursor cells in the bone marrow. Their main functions are to phagocytose cell debris and pathogens and to activate lymphocytes or other immune cells to respond to the pathogens in the form of fixed cells or free cells. At present, researches suggest that tumor cells have a mechanism of escaping macrophage phagocytosis. During the growth of tumor cells, specific proteins such as calreticulin are formed on the surface, exposing the identity of the tumor cells, such that the tumor cells are phagocytosed by the attracted macrophages. However, tumor cells with highly expressed CD47 are mistakenly recognized as normal cells by macrophages with SIRP$\alpha$ and thus escape macrophage phagocytosis, since the CD47-SIRP$\alpha$ pathway activates the inhibition of the macrophage phagocytosis (CD47 is upregulated on circulating hematopoietic stem cells and leukemia cells to avoid phagocytosis. Jaiswal S, Jamieson C H M, Pang W W, et al., Cell, 2009, 138(3) 271-285).

At present, researches suggest that anti-CD47 antibodies kill tumor cells primarily through two mechanisms. 1. Binding of anti-CD47 antibodies to CD47 blocks the CD47-SIRP$\alpha$ pathway, allowing macrophages' phagocytosis. 2. Anti-CD47 antibodies exert a tumor-killing effect through DC cells and CD8+ T cells. DC cells phagocytose tumor cells through the synergism between anti-CD47 antibodies and pro-phagocytic molecules such as calreticulin, and present tumor-associated antigens to CD8+ T cells, thereby exerting the specific killing effect of CD8+ T cells on tumors (CD47 blockade as another immune checkpoint therapy for cancer. Vonderheide R H. Nature Medicine, 2015, 21(10):1122). With the two mechanisms, it is suggested that anti-CD47 antibodies are very likely to have the ability to activate both non-specific immunity and specific immunity.

At present, anti-CD47 monoclonal antibody drugs have promising utility in a variety of applications and good efficacy in treating tumors, and can be used for treating various tumors. The anti-CD47 monoclonal antibody drug Hu5F9-G4 effectively inhibits the growth and metastasis of hematological malignancies and solid tumors in preclinical studies (Abstract PR13: The anti-CD47 antibody Hu5F9-G4 is a novel immune checkpoint inhibitor with synergistic efficacy in combination with clinically active cancer targeting antibodies [J] Chao M P, McKenna K M, Cha A, et al., 2016), wherein the amino acid sequence of the heavy chain is set forth in SEQ ID NO: 85, and the amino acid sequence of the light chain is set forth in SEQ ID NO: 86.

Therefore, the development of antibody drugs with high affinity for CD47, higher efficacy and fewer toxic side effects for treating tumors is of great significance.

**SUMMARY**

Mammalian cell expression systems were used to express recombinant human CD47 as an antigen to immunize mice, and hybridoma cells were obtained by fusion of mouse spleen cells and myeloma cells. The following hybridoma cell lines are obtained by screening a large number of the samples.

The inventors found that:

The hybridoma cell line LT012 can secrete a monoclonal antibody (named 6F7) capable of specifically binding to CD47, and the monoclonal antibody can compete with the receptor SIRPα ECD-hFc-Biotin for binding to CD47, effectively blocking the binding of SIRPα to CD47 and further promoting the phagocytosis of tumor cells by macrophages.

Furthermore, the inventors have prepared humanized versions of the monoclonal antibody 6F7 (named 6F7H1L1, 6F7H2L2 and 6F7H3L3).

The present invention is detailed below.

The amino acid sequences of the CDR regions of the antibodies are analyzed by technical means well known to those skilled in the art, for example, by VBASE2 database.

The antibodies 6F7, 6F7 H1L1, 6F7 H2L2 and 6F7 H3L3 disclosed herein share the same HCDR1-3 and LCDR1-3.

The 3 CDRs of the heavy chain variable region have the following amino acid sequences:

HCDR1: GYTFTSYW (SEQ ID NO: 5),
HCDR2: IDPSDSET (SEQ ID NO: 6),
HCDR3: ARLYRWYFDV (SEQ ID NO: 7);
the 3 CDRs of the light chain variable region have the following amino acid sequences:

LCDR1: EIVGTY (SEQ ID NO: 8),
LCDR2: GAS (SEQ ID NO: 9),
LCDR3: GQSYNFPYT (SEQ ID NO: 10).

In some embodiments, the antibodies disclosed herein are selected from the group consisting of:

an amino acid sequence of a heavy chain of 6F7 H1L1 (G1M) (SEQ ID NO: 59)
an amino acid sequence of a light chain of 6F7 H1L1 (G1M) (SEQ ID NO: 60)
an amino acid sequence of a heavy chain of 6F7 H2L2 (G1M) (SEQ ID NO: 61)
an amino acid sequence of a light chain of 6F7 H2L2 (G1M) (SEQ ID NO: 62)
an amino acid sequence of a heavy chain of 6F7 H3L3 (G1M) (SEQ ID NO: 63)
an amino acid sequence of a light chain of 6F7 H3L3 (G1M) (SEQ ID NO: 64)
an amino acid sequence of a heavy chain of 6F7 H1L1 (hG4) (SEQ ID NO: 65)
an amino acid sequence of a light chain of 6F7 H1L1 (hG4) (SEQ ID NO: 66)
an amino acid sequence of a heavy chain of 6F7 H2L2 (hG4) (SEQ ID NO: 67)
an amino acid sequence of a light chain of 6F7 H2L2 (hG4) (SEQ ID NO: 68)
an amino acid sequence of a heavy chain of 6F7 H3L3 (hG4) (SEQ ID NO: 69)
an amino acid sequence of a light chain of 6F7 H3L3 (hG4) (SEQ ID NO: 70)

One aspect of the present invention relates to an isolated polypeptide comprising sequences set forth in SEQ ID NOs: 5, 6 and 7, wherein the polypeptide, as part of an anti-human CD47 antibody, specifically binds to human CD47, the antibody further comprising sequences set forth in SEQ ID NOs: 8, 9 and 10.

One aspect of the present invention relates to an isolated polypeptide comprising sequences set forth in SEQ ID NOs: 8, 9 and 10, wherein the polypeptide, as part of an anti-human CD47 antibody, specifically binds to human CD47, the antibody further comprising sequences set forth in SEQ ID NOs: 5, 6 and 7.

One aspect of the present invention relates to an isolated polypeptide comprising a sequence selected from sequences set forth in SEQ ID NOs: 2, 12, 16 and 20 or a variant thereof, wherein the polypeptide, as part of an anti-human CD47 antibody, specifically binds to human CD47, the antibody further comprising a sequence selected from sequences set forth in SEQ ID NOs: 4, 14, 18 and 22 or a variant thereof, wherein the variant has at least 85%, preferably 86%, 87%, 88%, 89%, 90%, 91%, 92%, 93%, 94%, 95%, 96%, 97%, 98% or 99% or higher sequence identity to the corresponding sequence, or has one or more (preferably 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30) conservative amino acid mutations (preferably substitutions, insertions or deletions) compared to the corresponding sequence; or

one aspect of the present invention relates to an isolated polypeptide comprising a sequence selected from sequences set forth in SEQ ID NOs: 4, 14, 18 and 22 or a variant thereof, wherein the polypeptide, as part of an anti-human CD47 antibody, specifically binds to human CD47, the monoclonal antibody further comprising a sequence selected from sequences set forth in SEQ ID NOs: 2, 12, 16 and 20 or a variant thereof, wherein the variant has at least 85%, preferably 86%, 87%, 88%, 89%, 90%, 91%, 92%, 93%, 94%, 95%, 96%, 97%, 98% or 99% or higher sequence identity to the corresponding sequence, or has one or more (preferably 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30) conservative amino acid mutations (preferably substitutions, insertions or deletions) compared to the corresponding sequence.

In one embodiment of the present invention, the antigen-binding fragment is selected from Fab, Fab', F(ab')$_2$, Fd, Fv, dAb, Fab/c, complementarity determining region (CDR) fragment, single-chain antibody (e.g., scFv), bivalent antibody and domain antibody.

In one embodiment of the present invention, the antibody that specifically binds to CD47 is a humanized antibody, a chimeric antibody or a multispecific antibody (e.g., a bispecific antibody).

In one embodiment of the present invention, the antibody that specifically binds to CD47 binds to human CD47 protein with a KD less than about $10^{-5}$ M, e.g., less than about $10^{-6}$ M, $10^{-7}$ M, $10^{-8}$ M, $10^{-9}$ M or $10^{-10}$ M or less. Preferably, the KD is measured by a Fortebio system.

In one embodiment of the present invention, the antibody that specifically binds to CD47 binds to human CD47 protein with an $EC_{50}$ less than about 100 nM, e.g., less than about 10 nM, 1 nM, 0.9 nM, 0.8 nM, 0.7 nM, 0.6 nM, 0.5 nM, 0.4 nM, 0.3 nM, 0.2 nM or 0.1 nM or less. Specifically, the $EC_{50}$ is measured by indirect ELISA.

In one embodiment of the present invention, the antibody that specifically binds to CD47 comprises constant regions, and the constant regions are derived from species other than murine, e.g., from a human antibody, preferably from a human IgG, more preferably from IgG1 or IgG4.

In one embodiment of the present invention, the constant regions of the antibody that specifically binds to CD47 are humanized, e.g., the heavy chain constant region is Ig gamma-1 chain C region, more preferably Ig gamma-1 chain C region under GenBank ACCESSION No. P01857 (SEQ ID NO: 58), or is Ig gamma-4 chain C region, more preferably Ig gamma-4 chain C region under GenBank ACCESSION No. P01861.1 (SEQ ID NO: 56); the light chain constant region is Ig kappa chain C region, more preferably Ig kappa chain C region under GenBank ACCESSION No. P01834 (SEQ ID NO: 57). The antibodies disclosed herein use the following constant regions on the basis of the variable regions of 6F7 H1L1, 6F7 H2L2 and 6F7 H3L3: the heavy chain constant region is Ig gamma-1 chain C region under ACCESSION No. P01857 (SEQ ID NO: 58) or the heavy chain constant region is Ig gamma-4 chain C region under ACCESSION No. P01861.1 (SEQ ID NO: 56), and on this basis, S228P mutation is introduced to improve the stability; the light chain constant region is Ig kappa chain C region, ACCESSION No. P01834 (SEQ ID NO: 57). Another aspect of the present invention relates to an isolated polynucleotide encoding a polypeptide comprising sequences set forth in SEQ ID NOs: 5, 6 and 7, wherein the polypeptide, as part of an anti-human CD47 antibody, specifically binds to human CD47, the antibody further comprising sequences set forth in SEQ ID NOs: 8, 9 and 10.

One aspect of the present invention relates to an isolated polynucleotide encoding a polypeptide comprising sequences set forth in SEQ ID NOs: 8, 9 and 10, wherein the polypeptide, as part of an anti-human CD47 antibody, specifically binds to human CD47, the antibody further comprising sequences set forth in SEQ ID NOs: 5, 6 and 7.

One aspect of the present invention relates to an isolated polynucleotide encoding a polypeptide comprising a sequence selected from sequences set forth in SEQ ID NOs: 2, 12, 16 and 20 or a variant thereof, wherein the polypeptide, as part of an anti-human CD47 antibody, specifically binds to human CD47, the antibody further comprising a sequence selected from sequences set forth in SEQ ID NOs: 4, 14, 18 and 22 or a variant thereof,

wherein the variant comprises a sequence having at least 85%, preferably 86%, 87%, 88%, 89%, 90%, 91%, 92%, 93%, 94%, 95%, 96%, 97%, 98% or 99% or higher sequence identity to the corresponding sequence or having one or more (preferably 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30) conservative amino acid mutations (preferably substitutions, insertions or deletions) compared to the corresponding sequence; or
one aspect of the present invention relates to an isolated polynucleotide encoding a polypeptide comprising a sequence selected from sequences set forth in SEQ ID NOs: 4, 14, 18 and 22 or a variant thereof, wherein the polypeptide, as part of an anti-human CD47 antibody, specifically binds to human CD47, the antibody further comprising a sequence selected from sequences set forth in SEQ ID NOs: 2, 12, 16 and 20 or a variant thereof,
wherein the variant has at least 85%, preferably 86%, 87%, 88%, 89%, 90%, 91%, 92%, 93%, 94%, 95%, 96%, 97%, 98% or 99% or higher sequence identity to the corresponding sequence, or has one or more (preferably 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30) conservative amino acid mutations (preferably substitutions, insertions or deletions) compared to the corresponding sequence; specifically, the polynucleotide molecule comprises or consists of a nucleotide sequence set forth in SEQ ID NO: 1, SEQ ID NO: 11, SEQ ID NO: 15 or SEQ ID NO: 19, or a sequence having at least 85%, preferably 86%, 87%, 88%, 89%, 90%, 91%, 92%, 93%, 94%, 95%, 96%, 97%, 98% or 99% or higher sequence identity to the sequence.

Specifically, the polynucleotide molecule comprises or consists of a nucleotide sequence set forth in SEQ ID NO: 3, SEQ ID NO: 13, SEQ ID NO: 17 or SEQ ID NO: 21, or a sequence having at least 85%, preferably 86%, 87%, 88%, 89%, 90%, 91%, 92%, 93%, 94%, 95%, 96%, 97%, 98% or 99% or higher sequence identity to the sequence.

One aspect of the present invention further provides a hybridoma cell line selected from the hybridoma cell line LT012 under CCTCC NO. 2018135, and a monoclonal antibody produced by the hybridoma cell line.

The present invention further relates to the following aspects:

1. A pharmaceutical composition, preferably for treating a tumor, comprising a component A and a component B, wherein the component A is an antibody that specifically binds to CD47 or an antigen-binding fragment thereof;

the component B is selected from one or more of the following: a component B1, a component B2 and a component B3, wherein the component B1 is a bispecific antibody or an antigen-binding fragment thereof, the component B2 is an anti-tumor chemotherapeutic, and the component B3 is an anti-PD-1 monoclonal antibody or an antigen-binding fragment thereof;

preferably, the composition further comprises a component C, wherein the component C is an anti-tumor therapeutic agent, and the component C is different from component B;

for example, according to the Kabat, IMGT, Chothia or AbM numbering system, the antibody that specifically binds to CD47 comprises CDR sequences selected from those comprised in the following heavy chain variable regions and light chain variable regions:

(1) an HCDR1, an HCDR2 and an HCDR3 comprised in a heavy chain variable region set forth in SEQ ID NO: 2, and

an LCDR1, an LCDR2 and an LCDR3 comprised in a light chain variable region set forth in SEQ ID NO: 4; or

(2) an HCDR1, an HCDR2 and an HCDR3 comprised in a heavy chain variable region set forth in SEQ ID NO: 12, and

an LCDR1, an LCDR2 and an LCDR3 comprised in a light chain variable region set forth in SEQ ID NO: 14; or

(3) an HCDR1, an HCDR2 and an HCDR3 comprised in a heavy chain variable region set forth in SEQ ID NO: 16, and

an LCDR1, an LCDR2 and an LCDR3 comprised in a light chain variable region set forth in SEQ ID NO: 18; or

(4) an HCDR1, an HCDR2 and an HCDR3 comprised in a heavy chain variable region set forth in SEQ ID NO: 20, and

an LCDR1, an LCDR2 and an LCDR3 comprised in a light chain variable region set forth in SEQ ID NO: 22 (preferably, according to the IMGT numbering system, the antibody comprises:

an HCDR1 comprising or consisting of a sequence set forth in SEQ ID NO: 5 or a variant thereof, an HCDR2 comprising or consisting of a sequence set forth in SEQ ID NO: 6 or a variant thereof, and an HCDR3 comprising or consisting of a sequence set forth in SEQ ID NO: 7 or a variant thereof; and the antibody further comprises:

an LCDR1 comprising or consisting of amino acids set forth in SEQ ID NO: 8 or a variant thereof, an LCDR2 comprising or consisting of an amino acid sequence set forth in SEQ ID NO: 9 or a variant thereof, and

an LCDR3 comprising or consisting of a sequence set forth in SEQ ID NO: 10 or a variant thereof);

the bispecific antibody is an anti-PD-1/anti-CTLA4 antibody or an anti-PD-1/anti-VEGFA antibody or a combination of the two; the bispecific antibody comprises a first protein functional region and a second protein functional region, wherein the first protein functional region targets PD-1, and the second protein functional region targets CTLA4 or VEGFA; the first protein functional region is an immunoglobulin, and the second protein functional region is a single-chain antibody; or the first protein functional region is a single-chain antibody, and the second protein functional region is an immunoglobulin;

preferably, two single-chain antibody molecules (preferably, two identical single-chain antibody molecules) are linked to one immunoglobulin molecule; preferably, the immunoglobulin is of IgG1 subtype (preferably, human IgG1 subtype); preferably, the single-chain antibody is linked to the N terminus or C terminus of the heavy chain of the immunoglobulin, wherein the numbers of the first protein functional region and the second protein functional region are each independently 1, 2 or more; preferably, the first protein functional region is linked to the second protein functional region either directly or via a first linker fragment; and/or the heavy chain variable region of the single-chain antibody is linked to the light chain variable region of the single-chain antibody either directly or via a second linker fragment; the first linker fragment and the second linker fragment are identical or different; preferably, the linker fragment is (GGGGS)n, wherein n is a positive integer, or more preferably, n is 1, 2, 3, 4, 5 or 6; preferably, the first linker fragment and the second linker fragment comprise amino acid sequences independently selected from SEQ ID NO: 119 and SEQ ID NO: 120; more preferably, the first linker fragment and the second linker fragment comprise an amino acid sequence set forth in SEQ ID NO: 120;

when the bispecific antibody is an anti-PD-1/anti-CTLA4 antibody,

the first protein functional region comprises HCDR1-HCDR3 comprised in a heavy chain variable region set forth in SEQ ID NO: 87, and LCDR1-LCDR3 comprised in a light chain variable region set forth in SEQ ID NO: 88 (preferably, according to the IMGT numbering system, the first protein functional region comprises an HCDR1 comprising or consisting of a sequence set forth in SEQ ID NO: 89 or a variant thereof,

an HCDR2 comprising or consisting of a sequence set forth in SEQ ID NO: 90 or a variant thereof,

an HCDR3 comprising or consisting of a sequence set forth in SEQ ID NO: 91 or a variant thereof,

an LCDR1 comprising or consisting of amino acids set forth in SEQ ID NO: 92 or a variant thereof,

an LCDR2 comprising or consisting of an amino acid sequence set forth in SEQ ID NO: 93 or a variant thereof, and

an LCDR3 comprising or consisting of a sequence set forth in SEQ ID NO: 94 or a variant thereof);

the second protein functional region comprises HCDR1-HCDR3 in a heavy chain variable region set forth in SEQ ID NO: 95, and LCDR1-LCDR3 in a light chain variable region set forth in SEQ ID NO: 96 (preferably, according to the IMGT numbering system, the second protein functional region comprises an HCDR1 comprising or consisting of a sequence set forth in SEQ ID NO: 97 or a variant thereof,

an HCDR2 comprising or consisting of a sequence set forth in SEQ ID NO: 98 or a variant thereof,

an HCDR3 comprising or consisting of a sequence set forth in SEQ ID NO: 99 or a variant thereof,

an LCDR1 comprising or consisting of amino acids set forth in SEQ ID NO: 100 or a variant thereof,

an LCDR2 comprising or consisting of an amino acid sequence set forth in SEQ ID NO: 101 or a variant thereof, and

an LCDR3 comprising or consisting of a sequence set forth in SEQ ID NO: 102 or a variant thereof); or

when the bispecific antibody is an anti-PD-1/anti-VEGFA antibody,

the first protein functional region comprises HCDR1-HCDR3 comprised in a heavy chain variable region set forth in SEQ ID NO: 103, and LCDR1-LCDR3 comprised in a light chain variable region set forth in SEQ ID NO: 104 (preferably, according to the IMGT numbering system, the first protein functional region comprises an HCDR1 comprising or consisting of a sequence set forth in SEQ ID NO: 105 or a variant thereof,

an HCDR2 comprising or consisting of a sequence set forth in SEQ ID NO: 106 or a variant thereof,

an HCDR3 comprising or consisting of a sequence set forth in SEQ ID NO: 107 or a variant thereof,

an LCDR1 comprising or consisting of amino acids set forth in SEQ ID NO: 108 or a variant thereof,

an LCDR2 comprising or consisting of an amino acid sequence set forth in SEQ ID NO: 109 or a variant thereof, and

an LCDR3 comprising or consisting of a sequence set forth in SEQ ID NO: 110 or a variant thereof);

the second protein functional region comprises HCDR1-HCDR3 in a heavy chain variable region set forth in SEQ ID NO: 111, and LCDR1-LCDR3 in a light chain variable region set forth in SEQ ID NO: 112 (preferably, according to the IMGT numbering system, the second protein functional region comprises an HCDR1 comprising or consisting of a sequence set forth in SEQ ID NO: 113 or a variant thereof,

an HCDR2 comprising or consisting of a sequence set forth in SEQ ID NO: 114 or a variant thereof,

an HCDR3 comprising or consisting of a sequence set forth in SEQ ID NO: 115 or a variant thereof,

an LCDR1 comprising or consisting of amino acids set forth in SEQ ID NO: 116 or a variant thereof,

an LCDR2 comprising or consisting of an amino acid sequence set forth in SEQ ID NO: 117 or a variant thereof, and

an LCDR3 comprising or consisting of a sequence set forth in SEQ ID NO: 118 or a variant thereof);

wherein,

the anti-PD1 monoclonal antibody comprises HCDR1-HCDR3 comprised in a heavy chain variable region set forth in SEQ ID NO: 121, and LCDR1-LCDR3 comprised in a light chain variable region set forth in SEQ ID NO: 122 (preferably, according to the IMGT numbering system, the anti-PD1 monoclonal antibody comprises an HCDR1 comprising or consisting of a sequence set forth in SEQ ID NO: 123 or a variant thereof,

an HCDR2 comprising or consisting of a sequence set forth in SEQ ID NO: 124 or a variant thereof,

an HCDR3 comprising or consisting of a sequence set forth in SEQ ID NO: 125 or a variant thereof,

an LCDR1 comprising or consisting of amino acids set forth in SEQ ID NO: 126 or a variant thereof,

an LCDR2 comprising or consisting of an amino acid sequence set forth in SEQ ID NO: 127 or a variant thereof, and

an LCDR3 comprising or consisting of a sequence set forth in SEQ ID NO: 128 or a variant thereof);

wherein the variant comprises a sequence having at least 80%, 81%, 82%, 83%, 84%, 85%, 86%, 87%, 88%, 89%, 90%, 91%, 92%, 93%, 94%, 95%, 96%, 97%, 98%, 99% homology to the corresponding sequence, or the variant comprises a sequence having one or more (preferably 1, 2 or 3) conservative amino acid mutations (preferably substitutions, insertions or deletions) compared to the corresponding sequence.

2. The pharmaceutical composition according to item 1, wherein the antibody that specifically binds to CD47 further comprises framework regions (FRs) in the heavy chain variable region and framework regions (FRs) in the light chain variable region selected from the group consisting of the following:

(1) the framework regions (FRs) in the heavy chain variable region including an FR-H1, an FR-H2, an FR-H3 and an FR-H4, wherein the FR-H1 comprises or consists of an amino acid sequence set forth in SEQ ID NO: 23 or a variant thereof; the FR-H2 comprises or consists of an amino acid sequence set forth in SEQ ID NO: 24 or a variant thereof; the FR-H3 comprises or consists of an amino acid sequence set forth in SEQ ID NO: 25 or a variant thereof; the FR-H4 comprises or consists of an amino acid sequence set forth in SEQ ID NO: 26 or a variant thereof; the framework regions (FRs) in the light chain variable region including an FR-L1, an FR-L2, an FR-L3 and an FR-L4, wherein the FR-L1 comprises or consists of an amino acid sequence set forth in SEQ ID NO: 27 or a variant thereof; the FR-L2 comprises or consists of an amino acid sequence set forth in SEQ ID NO: 28 or a variant thereof; the FR-L3 comprises or consists of an amino acid sequence set forth in SEQ ID NO: 29 or a variant thereof; the FR-L4 comprises or consists of an amino acid sequence set forth in SEQ ID NO: 30 or a variant thereof;
(2) the framework regions (FRs) in the heavy chain variable region including an FR-H1, an FR-H2, an FR-H3 and an FR-H4, wherein the FR-H1 comprises or consists of an amino acid sequence set forth in SEQ ID NO: 31 or a variant thereof; the FR-H2 comprises or consists of an amino acid sequence set forth in SEQ ID NO: 32 or a variant thereof; the FR-H3 comprises or consists of an amino acid sequence set forth in SEQ ID NO: 33 or a variant thereof; the FR-H4 comprises or consists of an amino acid sequence set forth in SEQ ID NO: 34 or a variant thereof; the framework regions (FRs) in the light chain variable region including an FR-L1, an FR-L2, an FR-L3 and an FR-L4, wherein the FR-L1 comprises or consists of an amino acid sequence set forth in SEQ ID NO: 35 or a variant thereof; the FR-L2 comprises or consists of an amino acid sequence set forth in SEQ ID NO: 36 or a variant thereof; the FR-L3 comprises or consists of an amino acid sequence set forth in SEQ ID NO: 37 or a variant thereof; the FR-L4 comprises or consists of an amino acid sequence set forth in SEQ ID NO: 38 or a variant thereof;
(3) the framework regions (FRs) in the heavy chain variable region including an FR-H1, an FR-H2, an FR-H3 and an FR-H4, wherein the FR-H1 comprises or consists of an amino acid sequence set forth in SEQ ID NO: 39 or a variant thereof; the FR-H2 comprises or consists of an amino acid sequence set forth in SEQ ID NO: 40 or a variant thereof; the FR-H3 comprises or consists of an amino acid sequence set forth in SEQ ID NO: 41 or a variant thereof; the FR-H4 comprises or consists of an amino acid sequence set forth in SEQ ID NO: 42 or a variant thereof; the framework regions (FRs) in the light chain variable region including an FR-L1, an FR-L2, an FR-L3 and an FR-L4, wherein the FR-L1 comprises or consists of an amino acid sequence set forth in SEQ ID NO: 43 or a variant thereof; the FR-L2 comprises or consists of an amino acid sequence set forth in SEQ ID NO: 44 or a variant thereof; the FR-L3 comprises or consists of an amino acid sequence set forth in SEQ ID NO: 45 or a variant thereof; the FR-L4 comprises or consists of an amino acid sequence set forth in SEQ ID NO: 46 or a variant thereof;
(4) the framework regions (FRs) in the heavy chain variable region including an FR-H1, an FR-H2, an FR-H3 and an FR-H4, wherein the FR-H1 comprises or consists of an amino acid sequence set forth in SEQ ID NO: 47 or a variant thereof; the FR-H2 comprises or consists of an amino acid sequence set forth in SEQ ID NO: 48 or a variant thereof; the FR-H3 comprises or consists of an amino acid sequence set forth in SEQ ID NO: 49 or a variant thereof; the FR-H4 comprises or consists of an amino acid sequence set forth in SEQ ID NO: 50 or a variant thereof; the framework regions (FRs) in the light chain variable region including an FR-L1, an FR-L2, an FR-L3 and an FR-L4, wherein the FR-L1 comprises or consists of an amino acid sequence set forth in SEQ ID NO: 51 or a variant thereof; the FR-L2 comprises or consists of an amino acid sequence set forth in SEQ ID NO: 52 or a variant thereof; the FR-L3 comprises or consists of an amino acid sequence set forth in SEQ ID NO: 53 or a variant thereof; the FR-L4 comprises or consists of an amino acid sequence set forth in SEQ ID NO: 54 or a variant thereof,

wherein the variant comprises a sequence having at least 80%, preferably at least 81%, 82%, 83%, 84%, 85%, 86%, 87%, 88%, 89%, 90%, 91%, 92%, 93%, 94%, 95%, 96%, 97%, 98% or 99% homology to the corresponding sequence or having one or more (preferably 1, 2, 3, 4, 5, 6, 7, 8, 9 or 10) conservative amino acid mutations (preferably substitutions, insertions or deletions) compared to the corresponding sequence.

3. The pharmaceutical composition according to item 1 or 2, wherein the antibody that specifically binds to CD47 comprises:

(1) a heavy chain variable region comprising or consisting of:

an amino acid sequence set forth in SEQ ID NO: 2 or a variant thereof, and
a light chain variable region comprising or consisting of:
an amino acid sequence set forth in SEQ ID NO: 4 or a variant thereof;

(2) a heavy chain variable region comprising or consisting of:

an amino acid sequence set forth in SEQ ID NO: 12 or a variant thereof, and
a light chain variable region comprising or consisting of:
an amino acid sequence set forth in SEQ ID NO: 14 or a variant thereof;

(3) a heavy chain variable region comprising or consisting of:

an amino acid sequence set forth in SEQ ID NO: 16 or a variant thereof, and
a light chain variable region comprising or consisting of:
an amino acid sequence set forth in SEQ ID NO: 18 or a variant thereof; and

(4) a heavy chain variable region comprising or consisting of:

an amino acid sequence set forth in SEQ ID NO: 20 or a variant thereof, and
a light chain variable region comprising or consisting of:

an amino acid sequence set forth in SEQ ID NO: 22 or a variant thereof,
the first protein functional region of the anti-PD-1/anti-CTLA4 antibody comprises:

(1) a heavy chain variable region comprising or consisting of:

a sequence set forth in SEQ ID NO: 87 or a variant thereof, and
a light chain variable region comprising or consisting of:
a sequence set forth in SEQ ID NO: 88 or a variant thereof; and

(2) a heavy chain variable region comprising or consisting of:

a sequence set forth in SEQ ID NO: 129 or a variant thereof, and
a light chain variable region comprising or consisting of:

a sequence set forth in SEQ ID NO: 130 or a variant thereof;
the second protein functional region of the anti-PD-1/anti-CTLA4 antibody comprises:

(1) a heavy chain variable region comprising or consisting of:

a sequence set forth in SEQ ID NO: 95 or a variant thereof, and
a light chain variable region comprising or consisting of:
a sequence set forth in SEQ ID NO: 96 or a variant thereof;

(2) a heavy chain variable region comprising or consisting of:

a sequence set forth in SEQ ID NO: 133 or a variant thereof, and
a light chain variable region comprising or consisting of:
a sequence set forth in SEQ ID NO: 134 or a variant thereof;

(3) a heavy chain variable region comprising or consisting of:

a sequence set forth in SEQ ID NO: 135 or a variant thereof, and
a light chain variable region comprising or consisting of:
a sequence set forth in SEQ ID NO: 136 or a variant thereof;

(4) a heavy chain variable region comprising or consisting of:

a sequence set forth in SEQ ID NO: 137 or a variant thereof, and
a light chain variable region comprising or consisting of:
a sequence set forth in SEQ ID NO: 138 or a variant thereof; and

(5) a heavy chain variable region comprising or consisting of:

a sequence set forth in SEQ ID NO: 131 or a variant thereof, and
a light chain variable region comprising or consisting of:

a sequence set forth in SEQ ID NO: 132 or a variant thereof;
preferably, the immunoglobulin of the anti-PD-1/anti-CTLA4 antibody comprises a heavy chain having an amino acid sequence set forth in SEQ ID NO: 139, and targets PD-1;
the first protein functional region of the anti-PD-1/anti-VEGFA antibody comprises:

(1) a heavy chain variable region comprising or consisting of:

a sequence set forth in SEQ ID NO: 103 or a variant thereof, and
a light chain variable region comprising or consisting of:

a sequence set forth in SEQ ID NO: 104 or a variant thereof;
the second protein functional region of the anti-PD-1/anti-VEGFA antibody comprises:

(1) a heavy chain variable region comprising or consisting of:

a sequence set forth in SEQ ID NO: 111 (preferably encoded by a nucleotide sequence set forth in SEQ ID NO: 145) or a variant thereof, and
a light chain variable region comprising or consisting of:

a sequence set forth in SEQ ID NO: 112 (preferably encoded by a nucleotide sequence set forth in SEQ ID NO: 146) or a variant thereof;
preferably, the immunoglobulin of the anti-PD-1/anti-VEGFA antibody comprises a heavy chain having an amino acid sequence set forth in SEQ ID NO: 140, and targets VEGFA;
the anti-PD-1 monoclonal antibody comprises or consists of a heavy chain variable region set forth in SEQ ID NO: 121 and a light chain variable region set forth in SEQ ID NO: 122;
wherein the variant has at least 85%, preferably 86%, 87%, 88%, 89%, 90%, 91%, 92%, 93%, 94%, 95%, 96%, 97%, 98% or 99% or higher sequence identity to the corresponding sequence, or comprises an amino acid sequence having one or more (preferably 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30) conservative amino acid mutations (preferably substitutions, insertions or deletions) compared to the corresponding sequence.

4. The pharmaceutical composition according to any one of items 1-3, wherein the antibody that specifically binds to CD47 further comprises a heavy chain constant region and a light chain constant region, and the constant regions are derived from species other than murine, e.g., from a human antibody, preferably from a human IgG or IgM, and more preferably from IgG1; preferably, the heavy chain constant region is Ig gamma-1 chain C region, ACCESSION No. P01857 (SEQ ID NO: 58) or Ig gamma-4 chain C region, ACCESSION No. P01861.1 (SEQ ID NO: 56); the light chain constant region is Ig kappa chain C region, ACCESSION No. P01834 (SEQ ID NO: 57); preferably, the antibody that specifically binds to CD47 is secreted by a hybridoma cell line LT012 under CCTCC NO. 2018135.

5. The pharmaceutical composition according to any one of items 1-4, wherein, according to the EU numbering system, the antibody that specifically binds to CD47 and the immunoglobulin comprise a heavy chain constant region having mutations at any 1, 2 or 3 of positions 234, 235 and 237, and the affinity constant of the bispecific antibody for FcγRIIIa and/or C1q is reduced after the mutation as compared to that before the mutation; preferably, the affinity constant is measured by a Fortebio Octet system;

preferably, the antibody that specifically binds to CD47 comprises an L234A and/or L235A mutation according to the EU numbering system;
more preferably, the heavy chain constant region of the immunoglobulin has one of the following combinations of mutations:

L234A and L235A; or
L234A and G237A; or
L235A and G237A; or
L234A, L235A and G237A;
still more preferably,

according to the EU numbering system, the heavy chain constant region of the immunoglobulin further has one of the combinations of the following mutations:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

6. The pharmaceutical composition according to any one of items 1-5, wherein the antibody that specifically binds to CD47 comprises or consists of a heavy chain and a light chain selected from the group consisting of the following:

(1) a heavy chain set forth in SEQ ID NO: 59 and a light chain set forth in SEQ ID NO: 60;
(2) a heavy chain set forth in SEQ ID NO: 61 and a light chain set forth in SEQ ID NO: 62;
(3) a heavy chain set forth in SEQ ID NO: 63 and a light chain set forth in SEQ ID NO: 64;
(4) a heavy chain set forth in SEQ ID NO: 65 and a light chain set forth in SEQ ID NO: 66;
(5) a heavy chain set forth in SEQ ID NO: 67 and a light chain set forth in SEQ ID NO: 68; and
(6) a heavy chain set forth in SEQ ID NO: 69 and a light chain set forth in SEQ ID NO: 70;

the anti-PD-1/anti-CTLA4 antibody comprises or consists of a heavy chain and a light chain selected from the group consisting of the following:
a heavy chain set forth in SEQ ID NO: 78 (preferably encoded by a nucleotide sequence set forth in SEQ ID NO: 77) and a light chain set forth in SEQ ID NO: 80 (preferably encoded by a nucleotide sequence set forth in SEQ ID NO: 79);
the anti-PD-1/anti-VEGFA antibody comprises or consists of a heavy chain and a light chain selected from the group consisting of the following:

a heavy chain set forth in SEQ ID NO: 141 (preferably encoded by a nucleotide sequence set forth in SEQ ID NO: 142) and a light chain set forth in SEQ ID NO: 143 (preferably encoded by a nucleotide sequence set forth in SEQ ID NO: 144);
the anti-PD-1 monoclonal antibody comprises or consists of a heavy chain and a light chain selected from the group consisting of the following: a heavy chain set forth in SEQ ID NO: 82 (preferably encoded by a nucleotide sequence set forth in SEQ ID NO: 81) and a light chain set forth in SEQ ID NO: 84 (preferably encoded by a nucleotide sequence set forth in SEQ ID NO: 83).

7. The pharmaceutical composition according to any one of items 1-6, wherein the antigen-binding fragment is selected from Fab, Fab', F(ab')$_2$, Fd, Fv, dAb, Fab/c, complementarity determining region (CDR) fragment, single-chain antibody (e.g., scFv), bivalent antibody and domain antibody.

8. The pharmaceutical composition according to any one of items 1-7, wherein the antibody that specifically binds to CD47 is a humanized antibody, a chimeric antibody or a multispecific antibody (e.g., a bispecific antibody).

9. The pharmaceutical composition according to any one of items 1-8, wherein the antibody that specifically binds to CD47 binds to human CD47 protein with a KD less than about $10^{-5}$ M, e.g., less than about $10^{-6}$ M, $10^{-7}$ M, $10^{-8}$ M, $10^{-9}$ M, or $10^{-10}$ M or less, or the antibody that specifically binds to CD47 binds to human CD47 protein with an EC$_{50}$ less than about 100 nM, e.g., less than about 10 nM, 1 nM, 0.9 nM, 0.8 nM, 0.7 nM, 0.6 nM, 0.5 nM, 0.4 nM, 0.3 nM, 0.2 nM, 0.1 nM or less.

10. The pharmaceutical composition according to any one of items 1-9, wherein the antibody that specifically binds to CD47 is in the form of an antibody conjugate comprising the antibody that specifically binds to CD47 or the antigen-binding fragment thereof according to any one of items 1-9 and a conjugated moiety conjugated thereto, wherein the conjugated moiety is a purification tag (e.g., a His tag), a cytotoxic agent or a detectable label; preferably, the conjugated moiety is a radioisotope, a luminescent substance, a colored substance, an enzyme or polyethylene glycol; or

the antibody that specifically binds to CD47 is in the form of a multispecific antibody, preferably a bispecific antibody, comprising the antibody that specifically binds to CD47 or the antigen-binding fragment thereof according to any one of items 1-9, and an antibody against another antigen and/or another antigenic epitope or an antigen-binding fragment thereof; or the antibody that specifically binds to CD47 is in the form of a fusion protein comprising the antibody that specifically binds to CD47 or the antigen-binding fragment thereof according to any one of items 1-9.

11. The pharmaceutical composition according to any one of items 1-10, wherein the anti-tumor therapeutic agent is selected from one or more of the following: a tyrosine kinase inhibitor, a DNA polymerase inhibitor, an anti-human CD20 antibody, an anti-human PDL-1 antibody, an anti-human PD-1 antibody, an anti-human CTLA-4 antibody, a BCL-2 inhibitor, an anti-human EGFR antibody, an anti-human HER2 antibody, an anti-human HER3 antibody, a cyclin-dependent kinase inhibitor, an anti-human VEGFR2 antibody, an anti-human VEGF antibody, a proteasome inhibitor, an angiogenesis inhibitor, a rapidly accelerated fibrosarcoma (RAF) inhibitor, acalabrutinib, a bispecific antibody and a fusion protein drug; preferably, the therapeutic agent is cetuximab, obinutuzumab or rituximab; preferably, the anti-tumor chemotherapeutic is selected from one or more of the following: an alkylating agent, an anthracycline, an antimetabolite, an antibiotic, a plant-based and/or hormonal drug, a platinum-based drug (e.g., cisplatin, carboplatin and oxaliplatin), adriamycin, cyclophosphamide, a taxane (e.g., albumin-bound paclitaxel, liposome paclitaxel and docetaxel), etoposide, gemcitabine, pemetrexed, capecitabine, olaparib, rucaparib, niraparib, talazoparib, fluzoparib, vinca alkaloid, tamoxifen, megestrol, goserelin, asparaginase, a fluorouracil antineoplastic drug, azacitidine, cytarabine and cyclocytidine.

12. The pharmaceutical composition according to any one of items 1-11, wherein the pharmaceutical composition further comprises a pharmaceutically acceptable carrier and/or excipient.

13. The pharmaceutical composition according to any one of items 1-12, wherein, based on the mass of the antibody, the component A and the component B1 are in a mass ratio of (1:5)-(5:1), e.g., 1:5, 1:4, 1:3, 1:2, 1:1, 2:1, 3:1, 4:1 or 5:1;

the component A and the component B3 are in a mass ratio of (1:5)-(5:1), e.g., 1:5, 1:4, 1:3, 1:2, 1:1, 2:1, 3:1, 4:1 or 5:1;
or
the component B2 and the component A are in a mass ratio of 1:(1-1000), preferably 1:(5-500), and more preferably 1:(10-100);
preferably, the component A and the component B in the pharmaceutical composition are in a form suitable for administration by intravenous drip infusion, subcutaneous injection, intradermal injection, intravenous injection, intramuscular injection or intralesional injection.

14. A kit, preferably for treating a tumor, comprising a product A and a product B, wherein the product A comprises the antibody that specifically binds to CD47 or the antigen-binding fragment thereof as defined in any one of items 1-13;

the product B is selected from one or more of the following: a product B1, a product B2 and a product B3, wherein the product B1 is the bispecific antibody or the antigen-binding fragment thereof as defined in any one of items 1-13 (preferably, when the bispecific antibody is a combination of an anti-PD-1/anti-CTLA4 antibody and an anti-PD-1/anti-VEGFA antibody, the anti-PD-1/anti-CTLA4 antibody and the anti-PD-1/anti-VEGFA antibody are packaged either separately or together), the product B2 is the anti-tumor chemotherapeutic as defined in any one of items 1-13, and the product B3 is the anti-PD-1 monoclonal antibody or the antigen-binding fragment thereof as defined in any one of items 1-13, wherein the product B1, the product B2 and the product B3 are packaged either separately or together;
preferably, the kit further comprises a product C, wherein the product C is the anti-tumor therapeutic agent as defined in any one of items 1-13, and the product C is different from the product B;
preferably, the kit further comprises a package insert.

15. The kit according to item 14, wherein, based on the mass of the antibody, the product A and the product B1 are in a mass ratio of (1:5)-(5:1), e.g., 1:5, 1:4, 1:3, 1:2, 1:1,2:1,3:1,4:1 or 5:1;

the product A and the product B3 are in a mass ratio of (1:5)-(5:1), e.g., 1:5, 1:4, 1:3, 1:2, 1:1, 2:1, 3:1, 4:1 or 5:1; or
the anti-tumor chemotherapeutic is in a unit dose of 0.1-100 mg, 0.5-50 mg, 1-20 mg, 2-15 mg, 4-12 mg or 8-12 mg;
preferably, the product A, the product B or the product C in the kit is in a form suitable for administration by intravenous drip infusion, subcutaneous injection, intradermal injection, intravenous injection, intramuscular injection or intralesional injection.

16. A method for treating or preventing a tumor, comprising administering to a subject in need an effective amount of the component A and the component B as defined in any one of items 1-13, wherein preferably, the method further comprises administering to the subject in need an effective amount of the component C as defined in any one of items 1-13;

preferably, the component A, the component B and the component C are administered simultaneously or sequentially; more preferably, the component A, the component B and the component C are administered before or after a surgical treatment and/or before or after a radiotherapy;
preferably, when the component B is the component B1 and/or the component B3, the component A or the component B is administered at a unit dose of 0.1-100 mg, preferably 1-10 mg per kg body weight of the subject; or the component A or the component B is administered at a unit dose of 10-1000 mg, preferably 50-500 mg, 100-400 mg, 150-300 mg, 150-250 mg or 200 mg in each subject;
preferably, the component B2 is in a unit dose of 0.1-100 mg, 0.5-50 mg, 1-20 mg, 2-15 mg, 4-12 mg or 8-12 mg;
preferably, the dose is administered from twice daily to about once every other day, or once every 3 days, 4 days, 5 days, 6 days, 10 days, 1 week, 2 weeks, 3 weeks, 4 weeks, 5 weeks or 6 weeks;
preferably, the route of administration is intravenous drip infusion, subcutaneous injection, intradermal injection, intravenous injection, intramuscular injection or intralesional injection.

17. The pharmaceutical composition according to any one of items 1-13, the kit according to item 14 or 15, or the method according to item 16, wherein the tumor is preferably a tumor expressing CD47, and preferably a cancer; the cancer includes a solid tumor, a hematological tumor, lymphoma, blastoma, sarcoma, leukemia or lymphoid malignancy, and more preferably includes squamous cell carcinoma, myeloma, lung cancer, small cell lung cancer, non-small cell lung cancer, head and neck squamous cell carcinoma, glioma (e.g., neuroglioma and recurrent glioma), acute myelocytic leukemia, Hodgkin's lymphoma, non-Hodgkin's lymphoma, diffuse large B-cell lymphoma, follicular lymphoma, acute lymphocytic leukemia, acute myeloblastic leukemia, chronic lymphocytic leukemia, chronic myeloblastic leukemia, primary mediastinal large B-cell lymphoma, mantle cell lymphoma, small lymphocytic lymphoma, T cell/histiocyte-rich large B-cell lymphoma, multiple myeloma, myeloid leukemia-1 protein, relapsed and refractory peripheral T-cell lymphoma, myelodysplastic syndrome, anaplastic large cell lymphoma, mantle cell lymphoma, marginal zone lymphoma, myelofibrosis, polycythemia vera, bone marrow cancer, myeloproliferative disease, aggressive systemic mastocytosis, eosinophilia, dermatofibrosarcoma protuberans, chronic eosinophilic leukemia, gastrointestinal cancer, gastric adenocarcinoma or gastroesophageal junction adenocarcinoma, ovarian cancer, liver cancer, lymphocytic leukemia, large intestine cancer, endometrial cancer, prostate cancer, thyroid cancer, melanoma, chondrosarcoma, neuroblastoma, adenocarcinoma, pancreatic cancer, pancreatic ductal adenocarcinoma, glioblastoma multiforme, bone cancer, Ewing's sarcoma, cervical cancer, nasopharyngeal cancer, brain cancer, bladder cancer, breast cancer, triple negative breast cancer, intestinal cancer, rectal cancer, colorectal cancer, colon cancer, hepatocellular carcinoma, renal cell carcinoma, clear cell renal cell carcinoma, head and neck cancer, throat cancer, hepatobiliary cancer, central nervous system cancer, esophageal carcinoma, esophageal squamous cell carcinoma, malignant pleural mesothelioma, systemic light chain amyloidosis, lymphocytic lymphoma, myeloproliferative neoplasm, neuroendocrine tumor, Merkel cell carcinoma, testicular cancer and skin cancer, peritoneal cancer, fallopian tube cancer, urothelial cancer, microsatellite instability-high (MSI-H) or mismatch repair deficient (dMMR) cancer and mesothelioma.

18. A unit formulation, preferably for treating a tumor, comprising 1-10000 mg (preferably 10-1000 mg, and preferably 50-500 mg, 100-400 mg, 150-300 mg, 150-250 mg or 200 mg) of the component A and the component B as defined in any one of items 1-13,

wherein, when the component B is the component B1 and/or the component B3, the unit formulation comprises 1-10000 mg (preferably 1-1000 mg, and preferably 50-500 mg, 100-400 mg, 150-300 mg, 150-250 mg, 200 mg or 100 mg) of the component B as defined in any one of items 1-13;
when the component B is the component B2, the unit formulation comprises 0.1-100 mg, 0.5-50 mg, 1-20 mg, 2-15 mg, 4-12 mg, or 8-12 mg of the component B;
preferably, the unit formulation further comprises one or more of the components C as defined in any one of items 1-13;
wherein the component A, the component B and the component C are packaged separately.

19. A single dose unit, preferably for treating a tumor, comprising 0.1-10000 mg (preferably 1-1000 mg, and preferably 50-500 mg, 100-400 mg, 150-300 mg, 150-250 mg, 200 mg or 100 mg) of the component A and the component B as defined in any one of items 1-13,

wherein, when the component B is the component B1 and/or the component B3, the single dose unit comprises 0.1-10000 mg (preferably 1-1000 mg, and preferably 50-500 mg, 100-400 mg, 150-300 mg, 150-250 mg, 200 mg or 100 mg) of the component B as defined in any one of items 1-13;
when the component B is the component B2, the single dose unit comprises 0.1-100 mg, 0.5-50 mg, 1-20 mg, 2-15 mg, 4-12 mg, or 8-12 mg of the component B.

In a specific embodiment, the anti-tumor chemotherapeutic is selected from azacitidine, an anthracycline, cytarabine, gemcitabine and cyclocytidine.

The anti-human PD-1 antibody is selected from pembrolizumab, nivolumab, cemiplimab, toripalimab, sintilimab, camrelizumab and tislelizumab.

The anti-human CD20 antibody is selected from rituximab, obinutuzumab, BCD-132, H-02, B-001, IMM-0306, ACE-1755, IMM-03, JMT-601, TXB-4BC1 and GB-4542.

The anti-human EGFR antibody is selected from cetuximab, panitumumab, necitumumab, nepidermin, nimotuzumab, amivantamab, HS-627, amelimumab, depatuxizumab, FmAb-2, GC-1118A, imgatuzumab, MVC-101, SCT-200, QL-1203, tomuzotuximab, JMT-101, MCLA-158, QL-1105, SYN-004, MCLA-129, WBP-297, AM-105, BH-2922, BMX-002, CMAB-017, DF-203, GB-263, JZB-29, SAH-EJ1, SFR-9X0122, UBP-1215, ABX-901, MCLA-125, TXB-4BC2, 111-In-ch806, depatuxizumab mafodotin, DR-50201, DXL-1218, ENLS-1, FS-101 and GI-3000.

The BCL-2 inhibitor is selected from venetoclax.

The anti-HER2 or anti-HER3 antibody is selected from trastuzumab, trastuzumab emtansine, trastuzumab deruxtecan, margetuximab, pertuzumab, disitamab vedotin, U-31402, ISU-104, SIB-001, 9F7-F11, EV-20Sap, U-31402, ARX-788, BAT-8001, HL-02, TAA-013, trastuzumab duocarmazine, A-166, AU-101, AU-105, BPX-603, ISB-1302, KN-026, MB-103, MRG-002, zanidatamab, zenocutuzumab, ACE-1702, ALTP-7, B-002, BAT-8001, BAT-1006, BAY-2701439, BTRC-4017A, CAMH-2, cinrebafusp alfa, CT-0508, DP-303c, DX-126262, FS-102, FS-1502, GQ-1001, HS-630, LCB-14, M-802, MBS-301, MT-5111, NJH-395, PF-06804103, SBT-6050, SENL-006, SHRA-1201, SHRA-1811, TT-16, ZW-49 and LZM-006.

The anti-human VERFR2 antibody is selected from ramucirumab, AK109, VXM-01, AVI-3207, gentuximab, KD-035 and JY-025.

The taxane is selected from paclitaxel, albumin-bound paclitaxel, liposome paclitaxel and docetaxel.

The cyclin-dependent kinase inhibitor is selected from palbociclib, seliciclib, milciclib, lerociclib, abemaciclib, ebvaciclib, ribociclib succinate, trilaciclib, SHR-6390, alvocidib hydrochloride, AT-7519, AZD-4573, BEY-1107, BPI-1178, CT-7001, FCN-437c, FIT-039, NUV-422, PF-07104091, XZP-3287, zotiraciclib citrate, AGM-130, AUR-102, fadraciclib, LY-3405105, HS-10342, ON-123300, QHRD-107, TQ-05510, voruciclib, JS-101, XH-30002, AZ-5576, ETH-155008, JS-104 and RMC-4550.

The proteasome inhibitor is selected from bortezomib, carfilzomib and ixazomib. The tyrosine kinase inhibitor is selected from anlotinib, acalabrutinib, brigatinib, alectinib hydrochloride, ibrutinib, radotinib dihydrochloride, bosutinib monohydrate, ponatinib hydrochloride, crizotinib, ruxolitinib phosphate, nilotinib hydrochloride hydrate, dasatinib hydrate and imatinib mesylate.

The angiogenesis inhibitor antibody drug is selected from bevacizumab, ramucirumab and ranibizumab.

The rapidly accelerated fibrosarcoma (RAF) inhibitor antibody drug is selected from LY3009120, dabrafenib, vemurafenib and sorafenib.

The hormonal inhibitor is selected from dexamethasone, tamoxifen, toremifen, flutamide, nilutamidt, leuprolide, goserelin, buserelin, aminoglutethimide and formesfane.

The anti-human PD-1 antibody is selected from pembrolizumab, nivolumab, cemiplimab, toripalimab, sintilimab, camrelizumab, tislelizumab and penpulimab. The anti-human PDL-1 antibody is selected from atezolizumab, avelumab, durvalumab and AK105.

The anti-human CTLA-4 antibody is selected from ZW25, ipilimumab and tremelimumab.

The anti-human PD-1/CTLA-4 bispecific antibody is selected from cadonilimab, SI-B003, QL1706, XmAb-20717, KN046, MGD019 and MEDI5752.

The anti-human PD-1/VEGFA bispecific antibody is selected from AK112 (i.e., VP101(hG1DM)).

The platinum-based drug is selected from cisplatin (DDP), carboplatin (CBP) and oxaliplatin (L-OHP).

The anti-human PDL-1 antibody is selected from avelumab, atezolizumab, durvalumab, JS-003, CS-100L, LY-3300054>KD-033, CK-301, CCX-4503, CX-072, KN-035, HRP00052, HRP00049, FAZ-053, GR-1405, KD-005, HLX-20, KL-A167, CBT-502, STI-A1014, REMD-290, BGB-A333, BCD-135 and MCLA-145O.

The fusion protein drug is selected from aflibercept, AVID200, trebananib and M7824.

In one embodiment of the present invention, the drug is in a form suitable for injection, preferably in a form suitable for administration by subcutaneous injection, intradermal injection, intravenous injection, intramuscular injection or intralesional injection.

In the present invention, unless otherwise defined, the scientific and technical terms used herein have the meanings generally understood by those skilled in the art. In addition, the laboratory operations of cell culture, molecular genetics, nucleic acid chemistry and immunology used in the present invention are the routine operations widely used in the corresponding fields. Meanwhile, in order to better understand the present invention, the definitions and explanations of the relevant terms are provided below.

As used herein, the term "antigen-binding region" means a protein or a portion of a protein that specifically binds to a given antigen. For example, a portion of an antibody comprising amino acid residues that interact with an antigen and confer the antibody the specificity and affinity for the antigen is referred to as an "antigen-binding region". The antigen-binding region generally comprises one or more "complementarity determining regions" (CDRs). Certain antigen-binding regions further comprise one or more "framework" regions (FRs). CDRs are amino acid sequences that contribute to antigen-binding specificity and affinity.

As used herein, the term "antibody" refers to an intact immunoglobulin of any isotype or an antigen-binding fragment thereof that can compete with an intact antibody for specifically binding to a target antigen, and includes, for example, chimeric, humanized, fully humanized and bispecific antibodies or antigen-binding fragments thereof. Such "antibodies" are antigen-binding proteins. An intact antibody generally comprises at least two full-length heavy chains and two full-length light chains, but, in some cases, may comprise fewer chains, such as an antibody naturally existing in camelids that may comprise only a heavy chain. An antibody or an antigen-binding fragment thereof may be derived from a single source only, or may be "chimeric", i.e., different portions of the antibody may be derived from two different sources as further described below. An antibody or an antigen-binding fragment thereof may be produced in hybridomas by recombinant DNA technology, or by enzymatic or chemical cleavage of intact antibodies. Unless otherwise indicated, the term "antibody", in addition to antibodies comprising two full-length heavy chains and two full-length light chains, also includes derivatives, variants and fragments thereof.

As used herein, the term "antigen-binding fragment" (or abbreviated as "fragment") of an "antibody" or an "immunoglobulin chain" (heavy or light chain) comprises part of an antibody (whether obtained or synthesized) that lacks at least some of the amino acids present in the full length of the antibody but is capable of specifically binding to the antigen. Such fragments are biologically active as they specifically bind to a target antigen and can compete with other antibodies or antigen-binding fragments thereof for specifically binding to a given epitope. In one aspect, such fragments will retain at least one CDR present in the full-length light or heavy chain of the antibody, and in some embodiments, will comprise a single heavy and/or light chain or a portion thereof. Such biologically active fragments can be produced by recombinant DNA technology, or, for example, by enzymatic or chemical cleavage of intact antibodies. Immunologically functional immunoglobulin fragments include, but are not limited to, Fab, Fab', $F(ab')_2$, Fab/c, dAb, Fv, domain antibodies and single-chain antibodies, and can be derived from any mammalian source, including, but not limited to, human, mouse, rat, camelid and rabbit. It is further contemplated that a functional portion of an antibody disclosed herein, such as one or more CDRs, can be covalently bound to a second protein or a micromolecule to generate a therapeutic agent directed to a particular target in the body, thereby having bifunctional therapeutic properties or having an extended serum half-life, such as a fusion protein.

As used herein, the terms "antibody full-length chain", "full-length antibody", "intact antibody" and "whole antibody" are used interchangeably herein to refer to an antibody having a substantially similar structure to a natural antibody structure or having heavy chains in the Fc region as defined herein.

The term "light chain" includes full-length light chains and fragments thereof with sufficient variable region sequences to confer the binding specificity. The full-length light chain comprises a variable region domain $V_L$ and a constant region domain $C_L$. The variable region domain of the light chain is at the amino terminus of the polypeptide.

Light chains include kappa (κ) and lambda (λ) chains.

The term "heavy chain" includes full-length heavy chains and fragments thereof with sufficient variable region sequences to confer the binding specificity. The full-length heavy chain includes a variable region domain $V_H$ and 3 constant region domains $C_{H1}$, $C_{H2}$ and $C_{H3}$. The $V_H$ domain is at the amino terminus of the polypeptide, and the $C_H$ domains are at the carboxyl terminus, the $C_{H3}$ being closest to the carboxyl terminus of the polypeptide. The heavy chain may be of any isotype, including IgG (including IgG1, IgG2, IgG3 and IgG4 subtypes), IgA (including IgA1 and IgA2 subtypes), IgM and IgE.

As used herein, the term "Fab fragment" consists of one light chain, $C_{H1}$ and the variable region of one heavy chain. The heavy chain of an Fab molecule cannot form disulfide bonds with another heavy chain molecule.

As used herein, the term "Fc" region comprises two heavy chain fragments comprising the $C_{H1}$ and $C_{H2}$ domains of an antibody. The two heavy chain fragments are held together by two or more disulfide bonds and by the hydrophobic interaction of the $C_{H3}$ domains.

As used herein, the term "Fab' fragment" comprises one light chain and part of one heavy chain (containing the $V_H$ domain, the $C_{H1}$ domain, and part of the region between the $C_{H1}$ and $C_{H2}$ domains), such that interchain disulfide bonds can be formed between the two heavy chains of two Fab' fragments to form an $F(ab')_2$ molecule.

As used herein, the term "$F(ab')_2$ fragment" comprises two light chains and two heavy chains containing part of the constant region between the $C_{H1}$ and $C_{H2}$ domains, such that interchain disulfide bonds are formed between the two heavy chains. Thus, the $F(ab')_2$ fragment consists of two Fab' fragments held together by disulfide bonds between the two heavy chains.

As used herein, the term "Fv region" comprises the variable regions from the heavy and light chains, but lacks the constant regions.

As used herein, the term "Fd" fragment refers to an antibody fragment consisting of $V_H$ and $C_{H1}$ domains (Ward et al., Nature, 341:544-546 (1989)).

As used herein, the term "dAb" fragment consists of $V_H$ domains (Ward et al., Nature 341:544-546 (1989)).

As used herein, the term "Fab'-SH" is the designation herein for Fab', wherein one or more cysteine residues of the constant domain carry a free thiol group.

As used herein, the term "Fab/c" fragment is an intermediate formed by pepsin digestion of an immunoglobulin, and combines the advantages of Fab and Fc regions, i.e., strong diffusibility and low metabolic clearance *in vivo*, while retaining high affinity (Liu Jianjun, Chinese Journal of Cellular and Molecular Immunology, 1989(4):29-29).

As used herein, the term "single-chain antibody" is an Fv molecule in which the heavy and light chain variable regions are connected by a flexible linker to form a single polypeptide chain (which forms an antigen-binding region) (see, e.g., Bird et al., Science, 242:423-426 (1988), and Huston et al., Proc. Natl. Acad. Sci. USA., 90:5879-5883 (1988)). Single-chain antibodies are described in detail in International Patent Publication No. WO 88/01649 and U.S. Patent Nos. 4,946,778 and 5,260,203, the disclosures of which are incorporated herein by reference.

As used herein, the term "domain antibody" is an immunofunctional immunoglobulin fragment that comprises only the variable region of the heavy chain or the light chain, including multivalent domain antibodies or bivalent domain antibodies. In some cases, two or more $V_H$ regions are covalently linked by a peptide linker to generate a multivalent domain antibody (particularly a bivalent domain antibody). The two $V_H$ regions of the bivalent domain antibody may target the same or different antigens.

As used herein, the term "bivalent antigen-binding protein" or "bivalent antibody" comprises two antigen-binding sites. In some cases, the two binding sites have the same antigen specificity. The bivalent antibody may be bispecific.

As used herein, the term "multispecific antigen-binding protein" or "multispecific antibody" is an antigen-binding protein or antibody that targets more than one antigen or epitope.

As used herein, the term "bispecific", "dual-specificity" or "bifunctional" antigen-binding protein or antibody is a hybrid antigen-binding protein or an antibody having two different antigen-binding sites. A bispecific antibody is a multispecific antigen-binding protein or a multispecific antibody, and can be produced by a variety of methods, including but not limited to, fusion of hybridomas or linkage of Fab' fragments. See, e.g., Songsivilai and Lachmann, 1990, Clin. Exp. Immunol., 79:315-321; Kostelny et al., 1992, J. Immunol., 148:1547-1553. The two binding sites of a bispecific antigen-binding protein or antibody will bind to two different epitopes present in the same or different protein targets.

As used herein, the terms "mAb" and "monoclonal antibody" refer to an antibody or a fragment of an antibody that is derived from a group of highly homologous antibodies, i.e., from a group of identical antibody molecules except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using the hybridoma technique first reported by Kohler et al. (Nature, 256:495, 1975), but can also be obtained using recombinant DNA technology (see, e.g., U.S. Patent No. 4,816,567).

As used herein, the term "humanized antibody" refers to an antibody or an antibody fragment obtained when all or part of the CDR regions of a human immunoglobulin (receptor antibody) are replaced by the CDR regions of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, for example, Jones et al., Nature, 321:522-525 (1986); Reichmann et al., Nature, 332:323-329 (1988); Presta, Curr. Op. Struct. Biol., 2:593-596 (1992); and Clark, Immunol. Today, 21: 397-402 (2000).

As used herein, the term "epitope" refers to a site on an antigen to which an immunoglobulin or an antibody specifically binds. "Epitope" is also referred to in the art as an "antigenic determinant". The epitope or antigenic determinant generally consists of chemically active surface groups of molecules such as amino acids, carbohydrates or sugar side chains, and usually has specific three-dimensional structural characteristics and specific charge characteristics. For example, the epitope generally comprises at least 3, 4, 5, 6, 7, 8, 9, 10, 11, 12, 13, 14 or 15 consecutive or non-consecutive amino acids in a unique spatial conformation, which can be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

The terms "polypeptide" or "protein" are used interchangeably herein to refer to a polymer of amino acid residues. The term is also used to refer to an amino acid polymer in which one or more amino acid residues are analogs or mimetics of naturally existing amino acids, and to naturally existing amino acid polymers. The term may also include, for example, amino acid polymers that have been modified by addition of saccharide residues to form glycoproteins, or have been phosphorylated. Polypeptides and proteins can be produced by naturally existing cells and non-recombinant cells, or they may be produced by genetically engineered or recombinant cells, and comprise a molecule having the amino acid sequence of a native protein or a molecule having deletions, insertions and/or substitutions in one or more amino acids of the native sequence.

In some embodiments, the terms "polypeptide" and "protein" particularly include antibodies, such as anti-human CD47 antibodies (also referred to as CD47 antibodies), CD47-binding proteins, or variants thereof, e.g., antibodies or sequences having deletions, insertions, and/or substitutions of one or more amino acids.

The term "polypeptide fragment" refers to a polypeptide having an amino-terminal deletion, a carboxyl-terminal deletion, and/or an internal deletion as compared to a full-length protein. Such fragments may also comprise modified amino acids as compared to the full-length protein. In certain embodiments, such fragments are about 5 to 500 amino acids in length. For example, the fragment can be at least 5, 6, 8, 10, 14, 20, 50, 70, 100, 110, 150, 200, 250, 300, 350, 400 or 450 amino acids in length. Useful polypeptide fragments include immunologically functional fragments of antibodies, including binding domains. In the case of human CD47 antibodies, useful fragments include but are not limited to CDR regions, variable domains of heavy or light chains, parts of antibody chains, variable domains exactly comprising 2 CDRs, or the like.

A "derivative" of a polypeptide is a polypeptide (e.g., an antigen-binding protein or an antibody) that is chemically modified in other manners than insertion, deletion or substitution, e.g., by conjugation with another chemical moiety, e.g., a PEG-conjugated polypeptide.

In the present invention, letters before the position number represent amino acids before mutation, and letters after the position number represent amino acids after mutation, unless otherwise specified.

In some embodiments of the present invention, a disulfide bond is present between the VH and VL of the single-chain antibody. Methods for introducing a disulfide bond between the VH and VL of an antibody are well known in the art, see, for example, US 5,747,654; Rajagopal et al., Prot. Engin. 10(1997)1453-1459; Reiter et al., Nat. Biotechnol. 14(1996)1239-1245; Reiter et al., Protein Engineering 8(1995)1323-1331; Webber et al., Molecular Immunology 32(1995)249-258; Reiter et al., Immunity 2(1995)281-287; Reiter et al., JBC 269(1994)18327-18331; Reiter et al., Inter. J. of Cancer 58(1994)142-149; or Reiter et al., Cancer Res. 54(1994)2714-2718, which are incorporated herein by reference.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity ($K_D$) less than about $10^{-5}$ M, such as less than about $10^{-6}$ M, $10^{-7}$ M, $10^{-8}$ M, $10^{-9}$ M or $10^{-10}$ M or less.

As used herein, the term "$K_D$" refers to a dissociation equilibrium constant for a specific antibody-antigen interaction, which is used to describe the binding affinity between the antibody and the antigen. Among several parameters measured by molecular binding kinetics, the $K_D$ value is the dissociation equilibrium constant. In antibody drug research, it is the parameter characterizing the intensity of the affinity between an antibody of interest and the target antigen molecule, and is calculated by the formula: $KD = k_{dis}/k_{on}$. A smaller equilibrium dissociation constant indicates a more intensive antibody-antigen binding and a higher affinity between the antibody and the antigen. $k_{on}$ (association rate

constant) is the rate of antigen-antibody complex formation, and a smaller $k_{on}$ suggests a faster binding of an antibody to an antigen. $k_{dis}$ (dissociation rate constant) is the rate at which an antibody dissociates from an antigen-antibody complex, and a smaller $k_{dis}$ suggests a slower rate for the antibody dissociating from the antigen and a firmer binding between the antibody and the antigen. Generally, an antibody binds to an antigen (e.g., L1 protein) with a dissociation equilibrium constant ($K_D$) of less than about $10^{-5}$ M, e.g., less than about $10^{-6}$ M, $10^{-7}$ M, $10^{-8}$ M, $10^{-9}$ M or $10^{-10}$ M or less, for example, as measured by surface plasmon resonance (SPR) on a BIACORE system or by a Fortebio system. As used herein, the terms "monoclonal antibody" and "McAb" have the same meaning and can be used interchangeably; the terms "polyclonal antibody" and "PcAb" have the same meaning and can be used interchangeably; the terms "polypeptide" and "protein" have the same meaning and can be used interchangeably. Besides, as used herein, amino acids are generally represented by single-letter and three-letter abbreviations known in the art. For example, alanine can be represented by A or Ala.

As used herein, the terms "hybridoma" and "hybridoma cell line" can be used interchangeably, and when referring to the terms "hybridoma" and "hybridoma cell line", subclones and progeny cells of the hybridoma are also included.

As used herein, the terms "percent sequence identity" and "percent sequence homology" are used interchangeably.

As used herein, the terms "similarity", "sequence similarity" and "identity" refer to the correlation between the sequences of two or more protein or polypeptide molecules, as determined by aligning and comparing the sequences. "Percent identity" refers to the percentage of identical amino acid residues in the molecules compared, and can be calculated based on the size of the smallest molecule to be compared. For such calculations, gaps in the alignment (if any) must be addressed by a particular mathematical model or computer program (i.e., an "algorithm"). The term "substantial identity", when used for polypeptides, means that two peptide sequences, when optimally aligned, for example using the programs GAP or BESTFIT, using default gap weights provided by the programs, have at least 70%, 75% or 80% sequence identity, at least 90% or 95% sequence identity, or at least 97%, 98% or 99% sequence identity. In some cases, residue positions that are not identical differ in conservative amino acid substitutions. "Conservative amino acid substitution" is one in which the amino acid residue is substituted with another amino acid residue having a side chain R group that possesses similar chemical properties (e.g., charge or hydrophilicity). Generally, conservative amino acid substitutions will substantially retain the functions and properties of the protein. In cases where two or more amino acid sequences differ from each other by conservative substitutions, the percent sequence identity may be elevated to correct for the conservative nature of the substitution. Methods for making this adjustment are well known to those skilled in the art. See, e.g., Pearson, Methods Mol. Biol., 243:307-31 (1994). Examples of groups of amino acids having side chains with similar chemical properties include: 1) aliphatic hydroxyl side chain: glycine, alanine, valine, leucine and isoleucine, 2) aliphatic hydroxyl side chain: serine and threonine, 3) amide-containing side chain: asparagine and glutamine, 4) aromatic side chain: phenylalanine, tyrosine and tryptophan, 5) basic side chain: lysine, arginine and histidine, 6) acidic side chain: aspartic acid and glutamic acid, and 7) sulfur-containing side chain: cysteine and methionine. For example, the conservative amino acid substitution groups are valine-leucine-isoleucine-glycine-alanine, phenylalanine-tyrosine, threonine-serine, lysine-arginine, glutamic acid-aspartic acid and asparagine-glutamine.

Optionally, a conservative substitution is any change with a positive value in the PAM250 log-likelihood matrix disclosed in Gonnet et al., Science, 256:1443-45 (1992), which is incorporated herein by reference. A "moderately conservative" substitution is any change with a non-negative value in the PAM250 log-likelihood matrix.

The sequence identity of polypeptides is usually measured by a sequence analysis software. Protein analysis software aligns sequences using a measure of similarity assigned to different substitutions, deletions and other modifications (including conservative amino acid substitutions). For example, GCG, including programs such as "Gap" and "Bestfit", (using default parameters specified by the program) can be used to determine sequence homology or sequence identity between closely related polypeptides (e.g., homologous polypeptides from different biological species) or between a wild-type protein and its mutant protein. See, e.g., GCG Version 6.1 (University of Wisconsin, WI). Polypeptide sequences can also be compared using FASTA with default or recommended parameters. See GCG Version 6.10 FASTA (e.g., FASTA2 and FASTA3), which provides alignments for regions of optimal overlap between the challenge and query sequences and percent sequence identities (Pearson, Methods Enzymol., 183:63-98 (1990); Pearson, Methods Mol. Biol., 132:185-219 (2000)). Another preferred algorithm when comparing sequences with a database containing massive sequences from different organisms is the computer program BLAST, in particular, blastp or tblastn (using default parameters provided by the program). See, e.g., Altschul et al., Mol. Biol., 215:403-410 (1990); Al tschul et al., Nucleic Acids Res., 25:3389-402 (1997).

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants and ionic strength enhancers. For example, the pH regulators include, but are not limited to, phosphate buffer;

the surfactants include, but are not limited to, cationic, anionic or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop or delay the onset of the disease (e.g., a tumor); a therapeutically effective amount refers to an amount sufficient to cure or at least partially stop a disease and complications thereof in patients suffering from the disease.

The term "single dose unit" means a single pharmaceutical dosage form, such as an injection, e.g., packaged in an ampoule, comprising the anti-CD47 antibody and the combined therapeutic agents (e.g., the bispecific antibody, the anti-PD-1 monoclonal antibody and/or the anti-tumor chemotherapeutic) according to the present invention to be administered to a subject at time points of a regimen, preferably per kg body weight of the subject. In specific embodiments of the present invention, the regimen comprises, for example, administrations of the single dose unit according to a treatment cycle of from twice daily to about once every other day, or once every 3 days, 4 days, 5 days, 6 days, 10 days, 1 week, 2 weeks, 3 weeks, 4 weeks, 5 weeks or 6 weeks.

In the present invention, the terms "first" (e.g., first protein functional region or first linker fragment) and "second" (e.g., second protein functional region or second linker fragment) are used for distinguishing or clarity in expression and do not carry typical sequential meanings, unless otherwise specified.

A "therapeutically effective amount" or "therapeutically effective dose" of a drug or therapeutic agent is any amount of a drug that, when used alone or in combination with another therapeutic agent, protects a subject from the onset of a disease or promotes disease regression as evidenced by the reduction in the severity of disease symptoms, increase in the frequency and duration of disease symptom-free periods, or the prevention of damage or disability caused by the affliction of the disease. The ability of a therapeutic agent to promote disease regression can be evaluated using a variety of methods known to skilled practitioners, such as in a human subject in clinical trials, in an animal model system that predicts the efficacy in humans, or by determining the activity of the drug in an *in vitro* assay.

The "prophylactically effective amount" of a drug refers to any amount of a drug that inhibits the occurrence or recurrence of cancer when administered, alone or in combination with an antineoplastic agent, to a subject at risk of developing cancer (e.g., a subject having a premalignant condition) or a subject at risk of recurrence of cancer. In some embodiments, the prophylactically effective amount completely prevents the occurrence or recurrence of cancer. "Inhibiting" the occurrence or recurrence of cancer means reducing the possibility of the occurrence or recurrence of cancer or completely preventing the occurrence or recurrence of cancer. Compared with the prior art, the present invention has the following advantages: The anti-CD47 monoclonal antibody involved in the present invention can effectively block the binding of SIRP$\alpha$ to CD47 by specifically binding to CD47, thereby promoting the phagocytosis of tumor cells by macrophages, and can be used in combination with immune checkpoint inhibitors or other drugs, showing more excellent anti-tumor activity.

## BRIEF DESCRIPTION OF THE DRAWINGS

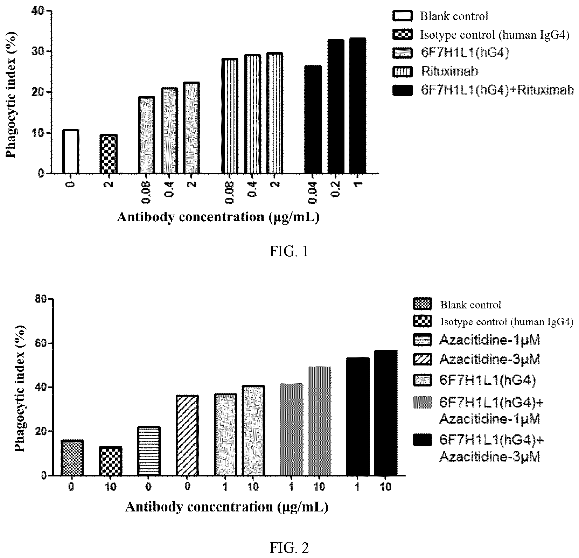

FIG. 1 shows the phagocytic index of HPMMs on Raji cells mediated by 6F7H1L1(hG4) in combination with rituximab.
FIG. 2 shows the phagocytic index of HPMMs on HL-60 cells mediated by 6F7H1L1(hG4) in combination with azacitidine.
FIG. 3 shows the activation of immune cells promoted by 6F7H1L1(hG4) in combination with PD-1/CTLA-4 bispecific antibody.
FIG. 4 shows the immune response of immune cells against human lung adenocarcinoma A549 cells promoted by 6F7H1L1(hG4) in combination with a PD-1 antibody.
FIG. 5 shows the immune response of immune cells against human ovarian cancer SK-OV-3 cells promoted by 6F7H1L1(hG4) in combination with a PD-1 antibody.
FIG. 6 shows the immune response of immune cells against human prostate cancer LNCAP cells promoted by 6F7H1L1(hG4) in combination with a PD-1 antibody.
FIG. 7 shows the phagocytic index of HPMMs on HT-29 cells mediated by 6F7H1L1(hG4) in combination with cetuximab.
FIG. 8 shows the phagocytic index of HPMMs on Raji cells mediated by 6F7H1L1(hG4) in combination with obinutuzumab.
FIG. 9 shows the efficacy of 6F7H1L1(hG4) in combination with anti-PD-1/anti-CTLA-4 bispecific antibody cadonilimab on a BALB/c-hPD1/hSIRP$\alpha$ mouse CT26-hCD47 tumor model.
FIG. 10 shows the body weight changes in the BALB/c-hPD1/hSIRP$\alpha$ mouse CT26-hCD47 tumor model receiving 6F7H1L1(hG4) in combination with anti-PD-1/anti-CTLA-4 bispecific antibody cadonilimab.

FIG. 11 shows the efficacy of 6F7H1L1(hG4) in combination with anti-PD-1/anti-VEGFA bispecific antibody VP101(hG1DM) on an SCID Beige mouse MDA-MB-231 tumor model.

FIG. 12 shows the body weight changes in the SCID Beige mouse MDA-MB-231 tumor model receiving 6F7H1L1(hG4) in combination with anti-PD-1/anti-VEGFA bispecific antibody VP101(hG1DM).

**Notes on the deposit of biological materials:**

Hybridoma cell line LT012 was deposited at China Center for Type Culture Collection (CCTCC) on Jun. 21, 2018 under CCTCC NO. C2018135, the depository address being Wuhan University, Wuhan, China, postal code: 430072.

**DETAILED DESCRIPTION**

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Guide to Molecular Cloning Experiments, authored by J. Sambrook et al., and translated by Huang Peitang et al., third edition, Science Press) or according to the product instruction. Reagents or instruments used are commercially available conventional products if the manufacturers thereof are not specified.

In the following examples of the present invention, BALB/C mice were purchased from Guangdong Medical Laboratory Animal Center.

In the following examples of the present invention, the isotype control antibodies used, i.e., hIgG1 and hIgG4, were antibodies targeting human anti-hen egg lysozyme (HEL), and the variable region sequences of these antibodies were from the study reported by Acierno et al., entitled "Affinity maturation increases the stability and plasticity of the Fv domain of anti-protein antibodies" (Acierno et al., J Mol biol., 2007; 374(1): 130-46, wherein the sequence of the heavy chain variable region is set forth in SEQ ID NO: 75, and the sequence of the light chain variable region is set forth in SEQ ID NO: 76). In the constant region fragment of hIgG1, the heavy chain constant region is Ig gamma-1 chain C region, ACCESSION No. P01857, and the light chain constant region is Ig kappa chain C region, ACCESSION No. P01834. In hIgG4, the heavy chain constant region is Ig gamma-4 chain C region, ACCESSION No. P01861.1 with S228P mutation introduced to improve the stability, and the light chain constant region is Ig kappa chain C region, ACCESSION No. P01834. Both hIgG1 and hIgG4 were prepared by Akeso Biopharma, Inc.

In the following examples of the present invention, the peripheral blood mononuclear cells (PBMCs) used were isolated and prepared by Akeso Biopharma, Inc. PBMCs were isolated from healthy human peripheral blood according to the manual of Ficoll-PaqueTM Plus reagent, and the isolated PBMCs were counted and frozen. The healthy human peripheral blood and PBMCs were isolated and extracted with the informed consent of the donors.

In the following examples of the present invention, the human peripheral monocyte-derived macrophages (HPMMs) used were derived from the PBMCs.

In the following examples of the present invention, the light and heavy chain sequences of anti-PD-1/CTLA-4 bispecific antibody cadonilimab, i.e., CP004(hG1TM) antibody, were derived from the sequences listed in WHO Drug Information, Proposed INN: List 124 (WHO Drug Information, 2020; 34(4):947-949, wherein the heavy chain sequence is set forth in SEQ ID NO: 78, and its coding nucleotide sequence is set forth in SEQ ID NO: 77; the light chain sequence is set forth in SEQ ID NO: 80, and its coding nucleotide sequence is set forth in SEQ ID NO: 79). The light and heavy chain sequences of anti-PD-1 antibody penpulimab were derived from the sequences listed in WHO Drug Information, Proposed INN: List 123 (WHO Drug Information, 2020; 34(2): 375-376, wherein the heavy chain sequence is set forth in SEQ ID NO: 82, and its coding nucleotide sequence is set forth in SEQ ID NO: 81; the light chain sequence is set forth in SEQ ID NO: 84, and its coding nucleotide sequence is set forth in SEQ ID NO: 83). The heavy chain sequence and the light chain sequence of the anti-PD-1/anti-VEGFA antibody are set forth in SEQ ID NO: 141 (encoded by a nucleotide sequence set forth in SEQ ID NO: 142) and SEQ ID NO: 143 (encoded by a nucleotide sequence set forth in SEQ ID NO: 144), respectively.

The method for preparing cadonilimab, anti-PD-1/anti-VEGFA antibody and penpulimab are as follows. The DNA sequence of the heavy chain-coding gene (including the constant region and the variable region) and the DNA sequence of the light chain-coding gene (including the constant region and the variable region) of the candidate antibodies were separately cloned into pUC57simple (supplied by GenScript) vectors to obtain plasmids pUC57simple-H and pUC57simple-L, respectively. The above plasmids were enzymatically digested (HindIII&EcoRI), and the heavy and light chains isolated by electrophoresis were separately subcloned into expression vector pcDNA3.1 (purchased from Invitrogen). The recombinant plasmids were extracted, and a combination of pcDNA3.1-H + pcDNA3.1-L was co-transfected into 293F cells. After 7 days of cell culture, the culture was centrifuged at high speed. The supernatant was concentrated, loaded onto a HiTrap MabSelect SuRe column, and purified using a protein purification liquid chromatography system (AKTA Purifier 10, GE). The purified sample, i.e., antibody cadonilimab, was added to both a reduced protein electrophoresis loading buffer and a non-reduced protein electrophoresis loading buffer, and then boiled for SDS-PAGE electrophoresis. By screening and confirming whether the protein conforms to the theoretical size, a purified antibody was obtained.

**Example 1: Preparation of anti-human CD47 antibody 6F7**

**1. Preparation of hybridoma cell line 6F7**

The antigen used for preparing the anti-CD47 antibody with the hybridoma cell line 6F7 was CD47 IgV TEV-His (including human CD47 mature peptide of positions 19-141 of GenbankID: NP 942088.1 and TEV (amino acid sequence: ENLYFQG, SEQ ID NO: 74)-his tag fusion protein, synthesized by Akeso Biopharma, Inc.) and 3T3-CD47 cells (NIH/3T3, manufacturer: ATCC, Cat. No: CRL-1658; the human CD47 mature peptide was transfected into the cells on the basis of NIH/3T3 to construct a cell line stably expressing 3T3-CD47). Spleen cells of immunized mice were fused with myeloma cells of the mice to prepare hybridoma cells. With CD47 IgV TEV-His and 3T3-CD47 cells separately taken as antigens, the hybridoma cells were screened by indirect ELISA to obtain hybridoma cells capable of secreting antibodies capable of specifically binding to CD47. The hybridoma cells obtained by ELISA screening were screened by competitive ELISA to obtain a hybridoma cell line capable of secreting a monoclonal antibody capable of competing for binding to CD47 IgV TEV-His with the receptor human SIRPαECD-hFc-Biotin (SIRPaECD refers to the extracellular region of SIRPα, positions 31-373 of protein GenBank ACCESSION No. NP_542970.1; hFc refers to a human IgG Fc purification label, specifically to Ig gamma-1 chain C region, positions 114-330 of GenbankID: P01857), which was then subjected to dilution cloning to obtain a stable hybridoma cell line. The hybridoma cell line was designated hybridoma cell line LT012, and the monoclonal antibody secreted by the cell line was designated 6F7.

Hybridoma cell line LT012 (CD47-6F7) was deposited at China Center for Type Culture Collection (CCTCC) on Jun. 21, 2018 under CCTCC NO. C2018135, the depository address being Wuhan University, Wuhan, China, postal code: 430072.

**2. Preparation of anti-CD47 antibody 6F7**

The cell lines LT011, LT012 and LT015 prepared above were separately cultured with a chemical defined medium (CD medium; containing 1% penicillin-streptomycin) in a 5% $CO_2$, 37 °C incubator. After 7 days, the supernatants were collected and purified by high-speed centrifugation and vacuum filtration through a microfiltration membrane and through a HiTrap protein A HP column to obtain antibody 6F7.

**Example 2: Sequence analysis of anti-CD47 antibody 6F7**

mRNA was extracted from the cell line LT012 cultured in Example 1 according to the method described in the manual of RNAprep pure Cell/Bacteria Kit (Tiangen, Cat. No. DP430).

cDNA was synthesized according to the manual of Invitrogen SuperScript® III First-Strand Synthesis System for RT-PCR and amplified by PCR.

The PCR-amplified products were directly subjected to TA cloning according to the manual of the pEASY-T1 Cloning Kit (Transgen CT101).

The TA-cloned products were directly sequenced, and the sequencing results are as follows:
The nucleic acid sequence of the heavy chain variable region is set forth in SEQ ID NO: 1 with a length of 351 bp.

The encoded amino acid sequence is set forth in SEQ ID NO: 2 with a length of 117 aa, and the sequences of heavy chain CDR1, CDR2 and CDR3 are set forth in SEQ ID NOs: 5, 6 and 7, respectively.

The nucleic acid sequence of the light chain variable region is set forth in SEQ ID NO: 3 with a length of 321 bp.

The encoded amino acid sequence is set forth in SEQ ID NO: 4 with a length of 107 aa, and the sequences of light chain CDR1, CDR2 and CDR3 are set forth in SEQ ID NOs: 8, 9 and 10, respectively.

**Example 3: Design and preparation of light and heavy chains of humanized anti-human CD47 antibodies 6F7 HIL1(hG4), 6F7 H2L2(hG4) and 6F7 H3L3(hG4)**

**1. Design of light and heavy chains of humanized anti-human CD47 antibodies 6F7 HIL1(hG4), 6F7 H2L2(hG4) and 6F7 H3L3(hG4)**

Based on the three-dimensional crystal structure of human CD47 protein (Hage T, Reinemer P, Sebald W., Crystals of a 1:1 Complex Between Human Interleukin-4 and the Extracellular Domain of Its Receptor Alpha Chain, Eur.

J. Biochem., 1998; 258(2):831-6.) and the sequence of antibody 6F7 obtained in Example 2, the variable region sequences of antibodies 6F7 H1L1, 6F7 H2L2 and 6F7 H3L3 were obtained by computer modeling and mutation design (antibody constant region sequences from NCBI database: the heavy chain constant region is Ig gamma-4 chain C region, ACCESSION No. P01861.1 with S228P mutation introduced to improve the stability on this basis; the light chain constant region is Ig kappa chain C region, ACCESSION No. P01834). The intact antibodies containing the constant regions were denoted as 6F7 H1L1(hG4), 6F7 H2L2(hG4) and 6F7 H3L3(hG4), respectively.

The designed variable region sequences are as follows:

(1) Heavy and light chain variable region sequences of humanized monoclonal antibody 6F7 H1L1

The nucleic acid sequence of the heavy chain variable region is set forth in SEQ ID NO: 11 with a length of 351 bp.

The encoded amino acid sequence is set forth in SEQ ID NO: 12 with a length of 117 aa, and the sequences of heavy chain CDR1, CDR2 and CDR3 are set forth in SEQ ID NOs: 5, 6 and 7, respectively.

The nucleic acid sequence of the light chain variable region is set forth in SEQ ID NO: 13 with a length of 321 bp.

The encoded amino acid sequence is set forth in SEQ ID NO: 14 with a length of 107 aa, and the sequences of light chain CDR1, CDR2 and CDR3 are set forth in SEQ ID NOs: 8, 9 and 10, respectively.

(2) Heavy and light chain variable region sequences of humanized monoclonal antibody 6F7 H2L2

The nucleic acid sequence of the heavy chain variable region is set forth in SEQ ID NO: 15 with a length of 351 bp.

The encoded amino acid sequence is set forth in SEQ ID NO: 16 with a length of 117 aa, and the sequences of heavy chain CDR1, CDR2 and CDR3 are set forth in SEQ ID NOs: 5, 6 and 7, respectively.

The nucleic acid sequence of the light chain variable region is set forth in SEQ ID NO: 17 with a length of 321 bp.

The encoded amino acid sequence is set forth in SEQ ID NO: 18 with a length of 107 aa, and the sequences of light chain CDR1, CDR2 and CDR3 are set forth in SEQ ID NOs: 8, 9 and 10, respectively.

(3) Heavy and light chain variable region sequences of humanized monoclonal antibody 6F7 H3L3

The nucleic acid sequence of the heavy chain variable region is set forth in SEQ ID NO: 19 with a length of 351 bp.

The encoded amino acid sequence is set forth in SEQ ID NO: 20 with a length of 117 aa, and the sequences of heavy chain CDR1, CDR2 and CDR3 are set forth in SEQ ID NOs: 5, 6 and 7, respectively.

The nucleic acid sequence of the light chain variable region is set forth in SEQ ID NO: 21 with a length of 321 bp.

The encoded amino acid sequence is set forth in SEQ ID NO: 22 with a length of 107 aa, and the sequences of light chain CDR1, CDR2 and CDR3 are set forth in SEQ ID NOs: 8, 9 and 10, respectively.

## 2. Preparation of humanized antibodies 6F7 HIL1(hG4), 6F7 H2L2(hG4) and 6F7 H3L3(hG4)

The heavy chain cDNA and light chain cDNA of 6F7H1L1(hG4), the heavy chain cDNA and light chain cDNA of 6F7H2L2(hG4), and the heavy chain cDNA and light chain cDNA of 6F7H3L3(hG4) were separately cloned into pUC57simple (provided by Genscript) vectors to obtain pUC57simple-6F7H1(**hG4**) and pUC57simple-6F7L1, pUC57simple-6F7H2(**hG4**) and pUC57simple-6F7L2, and pUC57simple-6F7H3(**hG4**) and pUC57simple-6F7L3, respectively. With reference to the standard techniques described in Molecular Cloning: A Laboratory Manual (Second Edition), the heavy and light chain full-length genes synthesized by EcoRI&HindIII digestion of genes were subcloned into expression vector pcDNA3.1 by restriction enzyme EcoRI&HindIII to obtain expression plasmids pcDNA3.1-6F7H1(**hG4**) and pcDNA3.1-6F7L1, pcDNA3.1-6F7H2(**hG4**) and pcDNA3.1-6F7L2, and pcDNA3.1-6F7H3(**hG4**) and pcDNA3.1-6F7L3, and the heavy and light chain genes of the recombinant expression plasmids were further sequenced. Then the designed gene combinations comprising corresponding light and heavy chain recombinant plasmids (pcDNA3.1-6F7H1(**hG4**)/pcDNA3.1-6F7L1, pcDNA3.1-6F7H2(**hG4**)/pcDNA3.1-6F7L2, and pcDNA3.1-6F7H3(**hG4**)/pcDNA3.1-6F7L3) were separately co-transfected into 293F cells, and the cultures were collected and purified. After the sequences were verified, endotoxin-free expression plasmids were prepared and were transiently transfected into HEK293 cells for antibody expression. The cultures were collected after 7 days, and subjected to affinity purification on a Protein A column (MabSelect SURE (GE)) to obtain humanized antibodies.

**Example 4: Design and preparation of light and heavy chains of humanized anti-human CD47 antibodies 6F7 H1L1(G1M), 6F7 H2L2(G1M) and 6F7 H3L3(GIM)**

**1. Design of light and heavy chains of humanized anti-human CD47 antibodies 6F7 H1L1(G1), 6F7 H2L2(G1) and 6F7 H3L3(G1)**

Based on the three-dimensional crystal structure of human CD47 protein (Hage T, Reinemer P, Sebald W., Crystals of a 1:1 Complex Between Human Interleukin-4 and the Extracellular Domain of Its Receptor Alpha Chain, Eur. J. Biochem., 1998; 258(2):831-6.) and the sequence of antibody 6F7 obtained in Example 2, the variable region sequences of antibodies 6F7 H1L1, 6F7 H2L2 and 6F7 H3L3 were obtained by computer modeling and mutation design (antibody constant region sequences from NCBI database: the heavy chain constant region is Ig gamma-1 chain C region, ACCESSION No. P01857; the light chain constant region is Ig kappa chain C region, ACCESSION No. P01834). For distinction from the humanized antibodies in Example 3, the humanized antibodies above were designated 6F7 H1L1(G1), 6F7 H2L2(G1) and 6F7 H3L3(G1), respectively.

The variable region sequences of the humanized antibodies 6F7 H1L1(G1), 6F7 H2L2(G1) and 6F7 H3L3(G1) designed in this example were identical to those of 6F7 H1L1(hG4), 6F7 H2L2(hG4) and 6F7 H3L3(hG4) in Example 3.

**2. Preparation of humanized antibodies 6F7 H1L1(G1), 6F7 H2L2(G1) and 6F7 H3L3(G1)**

On the basis of 6F7 H1L1(G1), 6F7 H2L2(G1) and 6F7 H3L3(G1), new humanized antibodies were obtained by introducing a leucine-to-alanine point mutation at position 234 (L234A, according to the EU numbering system) and a leucine-to-alanine point mutation at position 235 (L235A, according to the EU numbering system) in the hinge region of the heavy chain, and were designated 6F7 H1L1(G1M), 6F7 H2L2(G1M) and 6F7 H3L3(G1M), respectively.

The heavy chain cDNA and light chain cDNA of 6F7H1L1(G1M), the heavy chain cDNA and light chain cDNA of 6F7H2L2(G1M), and the heavy chain cDNA and light chain cDNA of 6F7H3L3(G1M) were separately cloned into pUC57simple (provided by Genscript) vectors to obtain pUC57simple-6F7H1(G1M) and pUC57simple-6F7L1, pUC57simple-6F7H2(G1M) and pUC57simple-6F7L2, and pUC57simple-6F7H3(G1M) and pUC57simple-6F7L3, respectively. With reference to the standard techniques described in Molecular Cloning: A Laboratory Manual (Second Edition), the heavy and light chain full-length genes synthesized by EcoRI&HindIII digestion of genes were subcloned into expression vector pcDNA3.1 by restriction enzyme EcoRI&HindIII to obtain expression plasmids pcDNA3.1-6F7H1(G1M) and pcDNA3.1-6F7L1, pcDNA3.1-6F7H2(G1M) and pcDNA3.1-6F7L2, and pcDNA3.1-6F7H3(G1M) and pcDNA3.1-6F7L3, and the heavy and light chain genes of the recombinant expression plasmids were further sequenced. Then the designed gene combinations comprising corresponding light and heavy chain recombinant plasmids (pcDNA3.1-6F7H1(G1M)/pcDNA3.1-6F7L1, pcDNA3.1-6F7H2(G1M)/pcDNA3 .1-6F7L2, and pcDNA3.1-6F7H3(G1M)/pcDNA3.1-6F7L3) were separately co-transfected into 293F cells, and the cultures were collected and purified. After the sequences were verified, endotoxin-free expression plasmids were prepared and were transiently transfected into HEK293 cells for antibody expression. The cultures were collected after 7 days, and subjected to affinity purification on a Protein A column (MabSelect SURE (GE)) to obtain humanized antibodies.

**Example 5: Phagocytosis of tumor cells by macrophages promoted by 6F7H1L1(hG4) in combination with rituximab or azacitidine**

Materials: Azacitidine (purchased from Baxter Oncology GmbH); Hu5F9-G4 (supplied by Akeso Biopharma, Inc.); Raji cells (Chinese Academy of Sciences Shanghai Branch); HL-60 cells (ATCC); RPMI 1640 (Gibco, Cat. No: 22400-105); pancreatin (0.25% Typsin-EDTA (1×)) (Gibco, Cat. No: 25200072); fetal bovine serum (FBS; Excell bio, Cat. No: FSP500); fluorescent dye 56-carboxyfluorescein diacetatesuccinimidyl ester (CFSE; Biolegend, Cat. No: 423801); recombinant human macrophage colony-stimulating factor (human M-CSF; PeproTech, Cat. No: 300-25); APC anti-human CD11c antibody (Biolegend, Cat. No: 301614); isotype control (human IgG4; supplied by Akeso Biopharma, Inc.); rituximab (Roche); BSA (Sigma, Cat. No: V900933-1KG); Ficoll paque plus (GE, Cat. No: 17-1440-02). Human peripheral monocyte-derived macrophages (HPMMs) were prepared by inducing PBMCs. Frozen PBMCs were conventionally thawed and incubated in an incubator overnight. The next day, PBMCs were collected, centrifuged at 170× g for 5 min, resuspended in a 1640 medium containing 2% FBS, and incubated in an incubator for 2 h. The supernatant was removed, and the cells were washed twice with PBS. 1640 complete medium (containing 10% FBS) and 100 ng/mL human M-CSF were added and the cells were inducted for 7 days. The medium was exchanged and 100 ng/mL human M-CSF was added on days 3 and 5. After the induction was completed on day 7, HPMMs were collected and centrifuged at 170× g for 5 min. The supernatant was discarded, and the cells were resuspended in 1640 complete medium (containing 10% FBS), with the cell density adjusted to $1 \times 10^{10}$ cells/mL. Then the cells were aliquoted into 1.5 mL EP tubes for later use.

**1. Phagocytosis of tumor cells by macrophages promoted by 6F7H1L1(hG4) in combination with rituximab**

Objective: In this study, the effects of 6F7H1L1(hG4) in combination with rituximab and 6F7H1L1(hG4) alone on the antibody-dependent cellular phagocytosis (ADCP) mediated by human Burkitt's lymphoma Raji cells were compared at the cytological level *in vitro*. In the assay system, HPMMs were used as the effector cells and Burkitt's lymphoma Raji cells were used as the target cells.

Procedures: Raji cells in the logarithmic phase were conventionally collected, centrifuged at $170\times$ g for 5 min, resuspended and then washed once with PBS. Raji cells were resuspended in CFSE (2.5 $\mu$M) to a staining density of $1\times10^7$ cells/mL and incubated in an incubator for 20 min. 6 mL of 1640 complete medium was added to stop staining. The cells were centrifuged at $170\times$ g for 5 min, and the supernatant was discarded. Then 1 mL of 1640 complete medium was added for resuspension, and the cells were incubated in an incubator for 10 min. The antibodies were diluted with 1640 complete medium to the working concentrations shown in the figure, and an isotype control antibody (an anti-HEL antibody, human IgG4) group was set. The antibodies and the target cells were added into EP tubes containing HPMMs at a volume ratio of 1:1 (the final volume was 100 $\mu$L, the effector-to-target ratio was $5\times10^4:1.5\times10^5$), and the mixtures were well mixed for resuspension and incubated in an incubator at 37 °C for 2 h. 800 $\mu$L of 1% PBSA (PBS containing 1% of BSA) at room temperature was added to each tube, and the system was centrifuged at $1200\times$ g for 5 min, and the supernatant was discarded. The cells were washed once with 800 $\mu$L of 1% PBSA. APC anti-human CD11c antibody (Biolegend, Lot No: 371506; diluted 400-fold with 1% PBSA) was added to the corresponding samples at 100 $\mu$L/sample for labeling, and the samples were incubated on ice for 40 min. 800 $\mu$L of 1% PBSA was added to each tube. The mixture was centrifuged at $1200\times$ g for 5 min and the supernatant was discarded. The cells were washed once with 800 $\mu$L of 1% PBSA. 200 $\mu$L of 1% PBSA was added to each tube for resuspension, and the cells were detected on an FACS Calibur flow cytometer. Macrophages in the system were APC-CD11b$^+$ positive, and macrophages involved in phagocytosis were APC-CD11b$^+$ and CFSE$^+$ dual positive. The phagocytic index was determined as the ratio of the number of dual positive cells to the number of APC positive cells, and the antibody-mediated ADCP activity was evaluated. The ADCP activity of each group, represented by P%, was calculated according to the following formula:

$$P\% = \frac{Number\ of\ macrophages\ involved\ in\ phagocytosis}{Total\ number\ of\ macrophages} \times 100\%$$

Results: In this study, the phagocytic activity (ADCP) mediated by 6F7H1L1(hG4) in combination with rituximab was determined using HPMMs as the effector cells and Raji cells as the target cells. The results, as shown in **Error! Citation source not found.,** demonstrated that the phagocytic indexes of rituximab and 6F7H1L1(hG4) were significantly higher than those of the blank control and the isotype control (human IgG4), indicating that rituximab and 6F7H1L1(hG4) have ADCP activity. The phagocytic indexes of 6F7H1L1(hG4) in combination with rituximab were significantly higher than those of 6F7H1L1(hG4), indicating that the ADCP activity of 6F7H1L1(hG4) in combination with rituximab is stronger than that of the monotherapy.

**2. Phagocytosis of tumor cells by macrophages promoted by 6F7H1L1(hG4) in combination with azacitidine**

Objective: In this study, the effects of 6F7H1L1(hG4) in combination with azacitidine and 6F7H1L1(hG4) alone on the antibody-dependent cellular phagocytosis (ADCP) mediated by acute myelocytic leukemia (AML) HL-60 cells were compared at the cytological level *in vitro*. In the assay system, HPMMs were used as the effector cells, and AML HL-60 cells treated with different concentrations of azacitidine were used as the target cells.

Procedures: HL-60 cells were conventionally collected and plated in a 6-well plate at $4\times10^5$ cells/2 mL of 1640 complete medium per well. Different concentrations of azacitidine (at working concentrations of 3 $\mu$M or 1 $\mu$M) were added. A blank HL-60 group was set. The cells were incubated in an incubator for 24 h. The next day, the treated and blank HL-60 cells were collected, centrifuged at $170\times$ g for 5 min, resuspended and counted. Then the cells were analyzed for viability and washed once with PBS. CFSE was diluted to 2.5 $\mu$M with PBS. HL-60 cells were resuspended in the diluted CFSE at a staining density of $1\times10^7$ cells/mL and incubated in an incubator for 20 min. 6 mL of 1640 complete medium (containing 10% FBS) was added to stop staining. The cells were centrifuged at $170\times$ g for 5 min, and the supernatant was discarded. Then 1 mL of 1640 complete medium was added for resuspension, and the cells were incubated in an incubator for 10 min. The antibodies were diluted with 1640 complete medium to 20 $\mu$g/mL and 2 $\mu$g/mL (the working concentrations were 10 $\mu$g/mL and 1 $\mu$g/mL), and an isotype control antibody (an anti-HEL antibody, human IgG4) group was set. The antibodies and the target cells were added into V-bottom plates containing HPMMs (the final volume was 100 $\mu$L, the effector-to-target ratio was $5\times10^4:1.5\times10^5$), and the mixtures were well mixed for resuspension and incubated in an incubator at 37 °C for 2 h. 150 $\mu$L of 1% PBSA (PBS containing 1% of BSA) at room temperature was added to each well, and the system was centrifuged at 1000× g for 5 min, and the supernatant was discarded. The cells were washed once with 200 μL of 1% PBSA. APC anti-human CD11c antibody (diluted 400-fold with 1% PBSA) was added to the corresponding samples at 100 μL/sample for labeling, and the samples were incubated on ice for 40 min. 150 μL of 1% PBSA was added to each well. The mixture was centrifuged at 1000× g for 5 min and the supernatant was discarded. The cells were washed once with 200 μL of 1% PBSA. 200 μL of 1% PBSA was added to each well for resuspension, and the cells were detected on an FACS Calibur flow cytometer. Macrophages in the system were APC-CD11b+ positive, and macrophages involved in phagocytosis were APC-CD11b+ and CFSE+ dual positive. The phagocytic index was determined as the ratio of the number of dual positive cells to the number of APC positive cells, and the antibody-mediated ADCP activity was evaluated. The ADCP activity of each group, represented by P%, was calculated according to the formula shown in (1) of this example.

Results: In this study, the ADCP activity assay of 6F7H1L1(hG4) in combination with azacitidine was conducted by using Hu5F9-G4 targeting CD47 as the positive control, HPMMs as the effector cells and HL-60 cells as the target cells. The results, as shown in **Error! Citation source not found.**, demonstrated that the phagocytic indexes of azacitidine, 6F7H1L1(hG4) and Hu5F9-G4 were significantly higher than those of the blank control and the isotype control (human IgG4), indicating that azacitidine, 6F7H1L1(hG4) and Hu5F9-G4 have pro-phagocytic activity. The phagocytic indexes of 6F7H1L1(hG4) and Hu5F9-G4 in combination with azacitidine were significantly higher than those of antibody monotherapies, indicating that the ADCP activities of 6F7H1L1(hG4) and Hu5F9-G4 in combination with azacitidine are stronger than those of antibody monotherapies. Compared with the control antibody Hu5F9-G4 for the same target, the ADCP activity of 6F7H1L1(hG4) in combination with azacitidine was comparable to that of Hu5F9-G4 in combination with azacitidine.

**Example 6: Activation of immune cells promoted by 6F7H1L1(hG4) in combination with PD-1/CTLA-4 bispecific antibody or PD-1 antibody**

Materials: PBMCs (isolated and prepared by Akeso Biopharma, Inc.); A549 cells (the Cell Center of the Chinese Academy of Sciences, Cat. No: KCB-200434YJ); SK-VO-3 cells (ATCC, Cat. No: HTB-77); HL-60 cells (ATCC, Cat. No: CCL-240); LNCAP (Chinese Academy of Sciences, Shanghai Institutes for Biological Sciences Cell Center); Raji-PDL1 cells (constructed by Akeso Biopharma, Inc., through constructing a PDL-1 lentiviral vector, infecting Raji cells after virus encapsulation and screening with blasticidin with conventional procedures); DMEM medium (Gibco, Cat. No: 11995-081); Trypsin-EDTA (0.25%); phenol red (Gibco, Cat. No: 25200072); RPMI 1640 medium (Gibco, Cat. No: 22400-105); Blasticidin (Gibco, Cat. No: R210-01); FBS (Excell bio, Cat. No: FSP500); SEB (*Staphylococcus aureus* enterotoxin; Dianotech, Cat. No: S010201); MMC (mitomycin C; Stressmarq, Cat. No: SIH-246); Ficoll-PaqueTM PLUS (GE Healthcare, Cat. No: 17-1440-02); human IL-2 ELISA KIT (Dakewe, Cat. No: 1110202), human IFN-γ ELISA KIT (Dakewe, Cat. No: 1110002); isotype control (human IgG1, or anti-HEL-isotype control).

**1. Activation of immune cells promoted by 6F7H1L1(hG4) in combination with PD-1/CTLA-4 bispecific antibody**

Objective: In this study, the activities of 6F7H1L1(hG4) in combination with a PD-1/CTLA-4 bispecific antibody (cadonilimab) and 6F7H1L1(hG4) alone in inducing the immune response of immune cells against human Burkitt's lymphoma Raji-PDL1 cells overexpressing human PDL-1 were compared at the cytological level *in vitro*.

Procedures: Raji-PDL1 cells were conventionally cultured in 1640 + 10% FBS complete medium. PBMCs were thawed and activated with 0.5 μg/mL SEB for two days. On the day of the experiment, Raji-PDL1 cells were treated with 2 μg/mL MMC for 1 h. SEB-activated PBMCs and MMC-treated Raji-PDL1 cells were collected, washed twice with PBS, resuspended in 1640 + 10% FBS complete medium and counted. Raji-PDL1 and PBMC cells were seeded on 96-well plates at $1 \times 10^5$ cells/well. The diluted antibody was added according to the study design. The mixture was mixed evenly and incubated in a 5% $CO_2$ incubator at 37 °C for 3 days. After 3 days, the cell culture supernatant was collected and analyzed for IL-2 and IFN-γ according to the manual of the ELISA KIT (Dakewe Biotech Co., Ltd.). The media in this study were all 10% FBS + 1640.

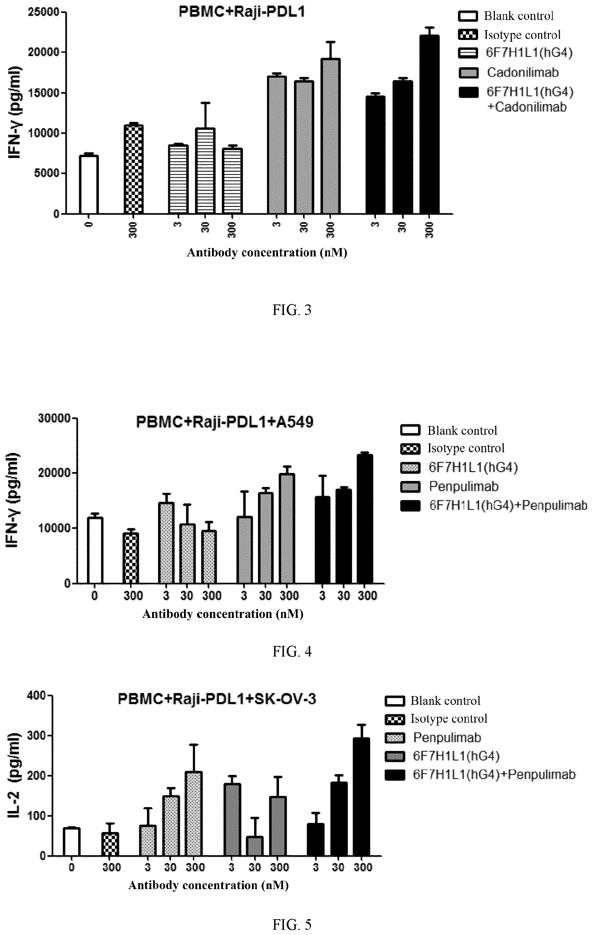

Results: The results, as shown in FIG. 3, demonstrated that 6F7H1L1(hG4) or PD-1/CTLA-4 bispecific antibody monotherapy can significantly stimulate the secretion of IFN-γ in PBMCs, while 6F7H1L1(hG4) in combination with the PD-1/CTLA-4 bispecific antibody showed superior activity to the monotherapies, i.e., enhances more significant immune response in immune cells against human Burkitt's lymphoma Raji-PDL1 cells.

**2. Activation of immune cells promoted by 6F7H1L1(hG4) in combination with PD-1 antibody**

**(1) Immune response of immune cells against human lung adenocarcinoma A549 cells promoted by 6F7H1L1(hG4) in combination with PD-1 antibody**

Objective: In this study, the activities of 6F7H1L1(hG4) in combination with a PD-1 antibody (penpulimab) and 6F7H1L1(hG4) alone in inducing the immune responses of immune cells against Raji-PDL1 cells and human lung adenocarcinoma A549 cells were compared at the cytological level *in vitro*.

Procedures: Raji-PDL1 cells were conventionally cultured in 1640 + 10% FBS complete medium, and A549 cells were conventionally cultured in DMEM + 10% FBS complete medium. PBMCs were thawed and activated with 0.5 $\mu$g/mL SEB for two days. On the day of the experiment, Raji-PDL1 cells were treated with 2 $\mu$g/mL MMC for 1 h. SEB-activated PBMCs and MMC-treated Raji-PDL1 cells were collected, washed twice with PBS, resuspended in 1640 + 10% FBS complete medium and counted. Raji-PDL1 and PBMC cells were seeded on 96-well plates at $1 \times 10^5$ cells/well. A549 cells in the logarithmic phase were collected and seeded on the 96-well plate at $5 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and analyzed for IL-2 and IFN-$\gamma$ according to the manual of the ELISA KIT. The media in this study were all 10% FBS + 1640.

Results: The results, as shown in FIG. 4, demonstrated that 6F7H1L1(hG4) or PD-1 antibody monotherapy can significantly stimulate the secretion of IFN-$\gamma$ by PBMC cells, while 6F7H1L1(hG4) in combination with the PD-1 antibody showed superior activity to the monotherapies, i.e., enhances more significant immune response in immune cells against human lung adenocarcinoma A549 cells.

**(2) Immune response of immune cells against human ovarian cancer SK-OV-3 cells promoted by 6F7H1L1(hG4) in combination with PD-1 antibody**

Objective: In this study, the activities of 6F7H1L1(hG4) in combination with a PD-1 antibody (penpulimab) and 6F7H1L1(hG4) alone in inducing the immune responses of immune cells against Raji-PDL1 cells and human ovarian cancer SK-OV-3 cells were compared at the cytological level *in vitro*.

Procedures: Raji-PDL1 cells were conventionally cultured in 1640 + 10% FBS complete medium, and SK-OV-3 cells were conventionally cultured in McCoy's 5A (Modified) Medium + 10% FBS complete medium. PBMCs were thawed and activated with 0.5 $\mu$g/mL SEB for two days. On the day of the experiment, Raji-PDL1 cells were treated with 2 $\mu$g/mL MMC for 1 h. SEB-activated PBMCs and MMC-treated Raji-PDL1 cells were collected, washed twice with PBS, resuspended in 1640 + 10% FBS complete medium and counted. Raji-PDL1 and PBMC cells were seeded on 96-well plates at $1 \times 10^5$ cells/well. SK-OV-3 cells in the logarithmic phase were collected and seeded on the 96-well plate at $5 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and analyzed for IL-2 and IFN-$\gamma$ according to the manual of the ELISA KIT. The media in this study were all 10% FBS + 1640.

Results: The results, as shown in FIG. 5, demonstrated that 6F7H1L1(hG4) or PD-1 antibody monotherapy can significantly stimulate the secretion of IL-2 by PBMC cells, while 6F7H1L1(hG4) in combination with the PD-1 antibody showed superior activity to the monotherapies, i.e., enhances more significant immune response in immune cells against human ovarian cancer SK-OV-3 cells.

**(3) Immune response of immune cells against human prostate cancer LNCAP cells promoted by 6F7H1L1(hG4) in combination with PD-1 antibody**

Objective: In this study, the activities of 6F7H1L1(hG4) in combination with a PD-1 antibody (penpulimab) and 6F7H1L1(hG4) alone in inducing the immune responses of immune cells against Raji-PDL1 cells and human prostate cancer LNCAP cells were compared at the cytological level *in vitro*.

Methodology: Raji-PDL1 cells were conventionally cultured in 1640 + 10% FBS complete medium, and LNCAP cells were conventionally cultured in RPMI-1640 + 10% FBS complete medium. PBMCs were thawed and activated with 0.5 $\mu$g/mL SEB for two days. On the day of the experiment, Raji-PDL1 cells were treated with 2 $\mu$g/mL MMC for 1 h. SEB-activated PBMCs and MMC-treated Raji-PDL1 cells were collected, washed twice with PBS, resuspended in 1640 + 10% FBS complete medium and counted. Raji-PDL1 and PBMC cells were seeded on 96-well plates at $1 \times 10^5$ cells/well. LNCAP cells in the logarithmic phase were collected and seeded on the 96-well plate at $5 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and analyzed for IL-2 and IFN-$\gamma$ according to the manual of the ELISA KIT. The media in this study were all 10% FBS + 1640.

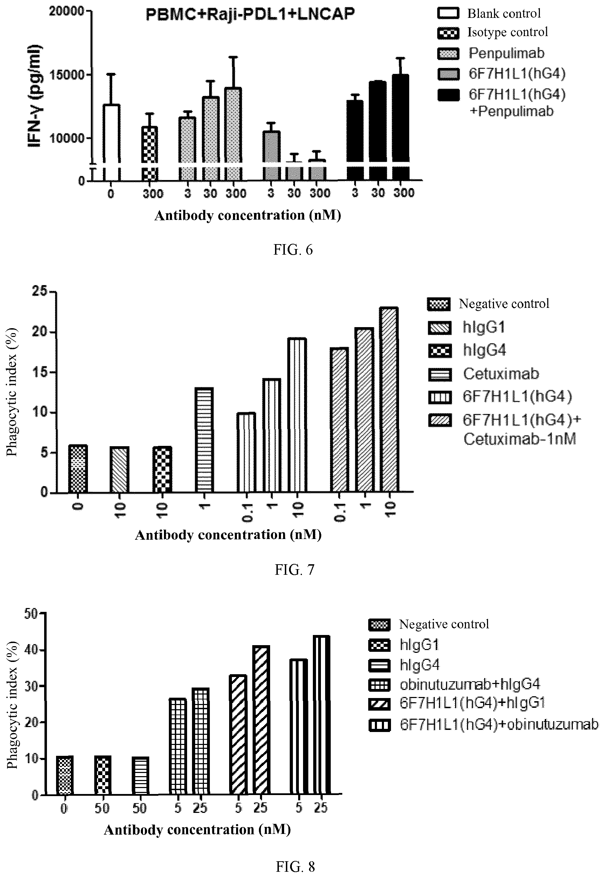

Results: The results, as shown in FIG. 6, demonstrated that 6F7H1L1(hG4) or PD-1 antibody monotherapy can significantly stimulate the secretion of IL-2 by PBMC cells, while 6F7H1L1(hG4) in combination with the PD-1 antibody showed superior activity to the monotherapies, i.e., enhances more significant immune response in immune cells against human prostate cancer LNCAP cells.

**Example 7: Phagocytosis of tumor cells by macrophages promoted by 6F7H1L1(hG4) in combination with cetuximab**

PBMCs (prepared by Akeso Biopharma, Inc., Lot No: 20200521-E) were thawed and incubated with 1640 complete medium + 10% FBS (Excell bio, Cat. No: FSP500, Lot No: 11I025) overnight. The next day, the medium was changed to 1640 complete medium + 2% FBS, and the cells were incubated with the medium for 2 h. The supernatant was removed, and the cells were washed twice with PBS and incubated in an incubator (90% 1640 + 10% FBS + M-CSF-100 ng/mL) to induce adult human macrophages (HPMMs, prepared by Akeso Biopharma, Inc.). The medium was exchanged and the treatment was given on days 3 and 5. The experiment was performed on day 7 of induction.

HT-29 cells (Chinese Academy of Sciences, Shanghai Institutes for Biological Sciences Cell Center, Cat. No: TCHu103) were conventionally collected, centrifuged at $170\times$ g for 5 min, resuspended and then counted. Then the cells were analyzed for viability and washed once with PBS. CFSE (Biolegend, Cat. No: 423801) was diluted to 2.5 $\mu$M with PBS. The cells were resuspended in a proper amount of the diluted CFSE to a staining density of $1\times10^7$ cells/mL and incubated in an incubator for 20 min. 6 mL of 1640 complete medium (containing 10% FBS) was added to stop staining. The cells were centrifuged at $170\times$ g for 5 min, and the supernatant was discarded. 1 mL of 1640 complete medium was added, and the cells were incubated in an incubator for 10 min, resuspended and counted. The number of cells was adjusted.

6F7H1L1(hG4) and cetuximab (prepared by Akeso Biopharma, Inc., Lot No: 20200917) were diluted to the desired concentrations with 1640 complete medium, and an isotype control antibody group was set. Human macrophages were collected and centrifuged at $7170\times$ g for 5 min, and the supernatant was discarded. The cells were counted, transferred to 1.5 mL EP tubes and centrifuged at $1200\times$ g for 5 min, and the supernatant was discarded. The tumor cells + antibody suspension (50 $\mu$L each) were added into the 1.5 mL EP tubes containing macrophages, and the mixtures were well mixed for resuspension and incubated in an incubator at 37 °C for 2 h. 800 $\mu$L of 1% PBSA at room temperature was added to each tube. The tubes were centrifuged at $1200\times$ g for 5 min, and the supernatant was discarded. The cells were washed once with 800 $\mu$L of PBSA. APC anti-mouse/human CD11b antibody (Biolegend, Cat. No: 101212, concentration: 0.2 mg/mL; diluted 500-fold with PBSA) was added to the corresponding samples at 100 $\mu$L/sample, and the mixtures were well mixed and incubated on ice for 40 min. 800 $\mu$L of 1% PBSA was added to each tube. The tubes were centrifuged at $1200\times$ g for 5 min, and the supernatant was discarded. The cells in each tube were washed once with 800 $\mu$L of PBSA. 200 $\mu$L of 1% PBSA was added to each tube for resuspension, and the cells were transferred to a flow cytometry tube and detected.

Results: In this study, the phagocytic activity (ADCP) mediated by 6F7H1L1(hG4) in combination with cetuximab was determined using HPMMs as the effector cells and HT29 cells as the target cells. The results, as shown in FIG. 7, demonstrated that the phagocytic indexes of cetuximab and 6F7H1L1(hG4) were significantly higher than those of the blank control and the isotype control (human IgG4), indicating that cetuximab and 6F7H1L1(hG4) have ADCP activity. The phagocytic indexes of 6F7H1L1(hG4) in combination with cetuximab were significantly higher than those of 6F7H1L1(hG4), indicating that the ADCP activity of 6F7H1L1(hG4) in combination with cetuximab is stronger than that of the monotherapy.

**Example 8: Phagocytosis of tumor cells by macrophages promoted by 6F7H1L1(hG4) in combination with obinutuzumab**

PBMCs (prepared by Akeso Biopharma, Inc., Lot No: 20201126-G) were thawed and incubated with 1640 complete medium + 10% FBS (Excell bio, Cat. No: FSP500, Lot No: 11I025) overnight. The next day, the medium was changed to 1640 complete medium + 2% FBS, and the cells were incubated with the medium for 2 h. The supernatant was removed, and the cells were washed twice with PBS and incubated in an incubator (90% 1640 + 10% FBS + M-CSF-100 ng/mL) to induce adult human macrophages (HPMMs). The medium was exchanged and the treatment was given on days 3 and 5. The experiment was performed on day 7 of induction.

Raji cells (purchased from ATCC) were conventionally collected and washed once with PBS. CFSE was diluted to 2.5 $\mu$M with PBS. The cells were resuspended in a proper amount of the diluted CFSE to a staining density of $1\times10^7$ cells/mL and incubated in an incubator for 20 min. 6 mL of 1640 complete medium (containing 10% FBS) was added to stop staining. The cells were centrifuged at $170\times$ g for 5 min, and the supernatant was discarded. 1 mL of 1640 complete medium was added, and the cells were incubated in an incubator for 10 min with the cell density adjusted to $3\times10^6$ cells/mL. The antibodies were diluted to the desired concentrations with 1640 complete medium, and isotype control antibody groups were set. Macrophages were collected and centrifuged at $170\times$ g for 5 min. The cells were adjusted to a density of $1\times10^6$ cells/mL, transferred to 96-well V-bottom plates and centrifuged at 750× g for 5 min, and the supernatant was discarded. The tumor cell + antibody suspension was added to the 96-well V-bottom plates containing macrophages, and the mixtures were well mixed for resuspension and incubated in an incubator at 37 °C for 2 h. 150 μL of 1% PBSA at room temperature was added to each well. The mixture was centrifuged at 750× g for 5 min, and the supernatant was discarded. The cells were washed once with 200 μL of PBSA. APC anti-mouse/human CD11b antibody (diluted 500-fold with PBSA) was added to the corresponding samples at 100 μL/sample, and the mixtures were well mixed and incubated on ice for 40 min. 150 μL of 1% PBSA was added to each well. The cells were centrifuged at 750× g for 5 min, and the supernatant was discarded. The cells in each well were washed once with 200 μL of PBSA. 200 μL of 1% PBSA was added to each tube for resuspension, and the cells were transferred to a flow cytometry tube and detected.

Results: In this study, the phagocytic activity (ADCP) mediated by 6F7H1L1(hG4) in combination with obinutuzumab was determined using HPMMs as the effector cells and Raji cells as the target cells. The results, as shown in FIG. 8, demonstrated that the phagocytic indexes of obinutuzumab and 6F7H1L1(hG4) were significantly higher than those of the blank control and the isotype controls (human IgG4 and human IgG1), indicating that obinutuzumab and 6F7H1L1(hG4) have ADCP activity. The phagocytic indexes of 6F7H1L1(hG4) in combination with obinutuzumab were higher than those of 6F7H1L1(hG4), indicating that the ADCP activity of 6F7H1L1(hG4) in combination with obinutuzumab is stronger than that of the monotherapy.

**Example 9: Effective anti-tumor treatment with 6F7H1L1(hG4) in combination with anti-PD-1/anti-CTLA-4 bispecific antibody**

In order to determine the tumor inhibitory activity *in vivo* of 6F7H1L1(hG4) antibody in combination with anti-PD-1/anti-CTLA-4 bispecific antibody cadonilimab, female BALB/c-hPD1/hSIRPα mice aged 6.43-8.43 weeks (purchased from GemPharmatech Co., Ltd.) were grafted subcutaneously with CT26-hCD47 cells (mouse colon cancer cells, purchased from GemPharmatech Co., Ltd.). When the mean tumor volume reached 80-120 mm$^3$, the mice were randomized into 4 groups of 6 by tumor volume. The day of grouping was defined as D0, and dosing was started on the day of grouping D0. The regimen of the combination therapy group is as follows: the drugs were formulated separately and administered sequentially (no certain order or time interval was required, and one treatment should be given after the completed administration of the other). The modeling and specific regimen are shown in Table 1. After the administration, the length and width of tumors in each group were measured, and the tumor volume was calculated. The data were subjected to an independent sample T-test, SPSS 17.0 *P <0.05, **P<0.01.

Table 1: Regimen for treating CT26-hCD47 xenograft tumor BALB/c-hPD1/hSIRPα mouse model with 6F7H1L1(hG4) antibody in combination with anti-PD-1/anti-CTLA-4 bispecific antibody cadonilimab

| Group | Number | Grafting | Administration |
|---|---|---|---|
| Isotype control 5 mg/kg | 6 | | Isotype control antibody hIgG4, intraperitoneally administered twice per week for 4 weeks, 7 doses |
| 6F7H1L1(hG4) 5mg/kg | 6 | | 6F7H1L1(hG4), 5 mg/kg; intraperitoneally administered twice per week for 4 weeks, 7 doses. |
| Cadonilimab 0.5mg/kg | 6 | CT26-hCD47, 5×10$^5$ cells, BALB/c-hPD1/hSIRPα mice, SC | Cadonilimab (prepared by Akeso Biopharma, Inc., Lot No: B104C20191206), 0.5 mg/kg; intraperitoneally administered twice per week for 4 weeks, 7 doses. |
| 6F7H1L1(hG4) 5mg/kg, Cadonilimab 0.5mg/kg | 6 | | 6F7H1L1(hG4), 5 mg/kg; cadonilimab, 0.5 mg/kg; intraperitoneally administered twice per week for 4 weeks, 7 doses. |

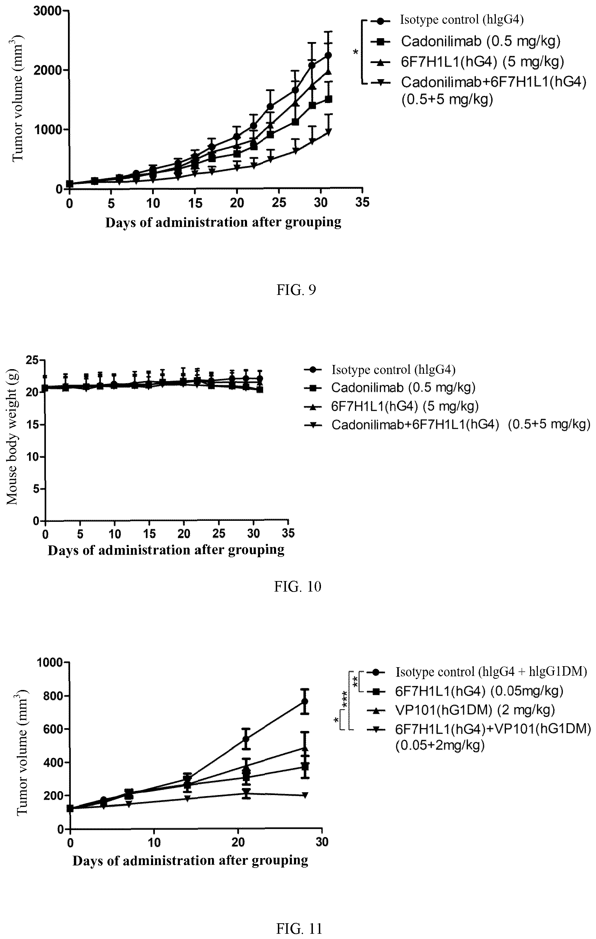

The results are shown in FIG. 9. The results showed that compared with the isotype control antibody hIgG4, both 6F7H1L1(hG4) and PD-1/CTLA-4 bispecific antibody cadonilimab can effectively inhibit the growth of tumors in mice, and the 6F7H1L1(hG4) + cadonilimab combination therapy group showed synergic anti-tumor efficacy in the model and was superior to the monotherapy groups in inhibiting tumors.

In addition, as shown in FIG. 10, both 6F7H1L1(hG4) and cadonilimab were well tolerated by the tumor-bearing mice, either alone or in combination, and no effect on the body weight of the tumor-bearing mice was found in the groups.

**Example 10: Effective anti-tumor treatment with 6F7H1L1(hG4) in combination with anti-PD-1/anti-VEGFA bispecific antibody**

In order to determine the tumor inhibitory activity *in vivo* of 6F7H1L1(hG4) antibody in combination with anti-PD-1/anti-VEGFA bispecific antibody VP101(hG1DM), SCID Beige mice aged 5-7 weeks (purchased from Charles River, Beijing) were grafted subcutaneously with MDA-MB-231 cells (purchased from ATCC). When the mean tumor volume reached 120-150 mm$^3$, the mice were randomized into 4 groups of 6 by tumor volume. The day of grouping was defined as D0. Dosing was started on the day of grouping D0, and the mice were injected intraperitoneally with activated PBMCs. The regimen of the combination therapy group is as follows: the drugs were formulated separately and administered sequentially (no certain order or time interval was required, and one treatment should be given after the completed administration of the other). The modeling and specific regimen are shown in Table 2. After the administration, the length and width of tumors in each group were measured, and the tumor volume was calculated. The data were subjected to a one-way analysis of variance (Bonferroni post hoc test), $^*P < 0.05$, $^{**}P < 0.01$.

Table 2: Regimen for treating MDA-MB-231 xenograft tumor SCID Beige mouse model with 6F7H1L1(hG4) antibody in combination with anti-PD-1/anti-VEGFA bispecific antibody VP101(hG1DM)

| Grouping | Number | Grafting | Administration |
|---|---|---|---|
| Isotype control | 6 | MDA-MB-231, | Isotype control antibody hIgG4, 0.05 |
| | | 8×10$^6$ cells, SCID Beige mice, SC; on the day of grouping, the mice were grafted with 4×10$^6$ CD3 antibody-activated human PBMCs via intraperitoneal injection | mg/kg; intraperitoneally administered twice per week for 4 weeks; Isotype control antibody hIgG1DM, 3.75 mg/kg; intraperitoneally administered once per week for 4 weeks |
| 6F7H1L1 (hG4) 0.05mg/kg | 6 | | 6F7H1L1(hG4), 0.05 mg/kg; intraperitoneally administered twice per week for 4 weeks |
| VP101 (hG1DM) 2mg/kg | 6 | | VP101(hG1DM) (prepared by Akeso Biopharma, Inc., Lot No: B112C20201205), 2 mg/kg; intraperitoneally administered once per week for 4 weeks |
| 6F7H1L1 (hG4) 0.05mg/kg, VP101 (hG1DM) 2mg/kg | 6 | | 6F7H1L1(hG4), 0.05 mg/kg; intraperitoneally administered twice per week for 4 weeks; VP101(hG1DM), 2 mg/kg; intraperitoneally administered once per week for 4 weeks |

The results are shown in FIG. 11. The results showed that compared with the isotype control group, both 6F7H1L1(hG4) and VP101(hG1DM) can effectively inhibit the growth of tumors in mice, and the 6F7H1L1(hG4) + VP101(hG1DM) combination therapy group showed synergistic anti-tumor efficacy on the model and was superior to the monotherapy groups in inhibiting tumors.

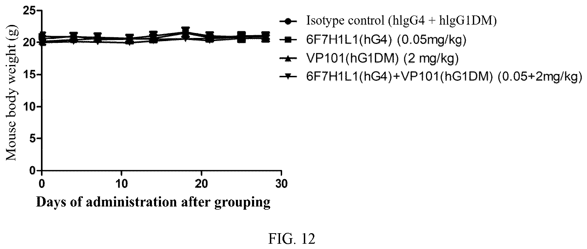

In addition, as shown in FIG. 12, both 6F7H1L1(hG4) and VP101(hG1DM) were well tolerated by the tumor-bearing mice, either alone or in combination, and no effect on the body weight of the tumor-bearing mice was found in the groups.

The embodiments of the present invention have been described above in detail, but the present invention is not limited to the embodiments. Those skilled in the art can make various equivalent modifications or replacements without departing from the spirit of the present invention. These equivalent modifications or replacements shall fall within the scope defined by the claims of the present application.

**Claims**

1.  A pharmaceutical composition, preferably for treating a tumor, comprising a component A and a component B, wherein the component A is an antibody that specifically binds to CD47 or an antigen-binding fragment thereof;

    the component B is selected from one or more of the following: a component B1, a component B2 and a component B3, wherein the component B1 is a bispecific antibody or an antigen-binding fragment thereof, the component B2 is an anti-tumor chemotherapeutic, and the component B3 is an anti-PD-1 monoclonal antibody or an antigen-binding fragment thereof;
    preferably, the composition further comprises a component C, wherein the component C is an anti-tumor therapeutic agent, and the component C is different from component B;
    for example, according to the Kabat, IMGT, Chothia or AbM numbering system, the antibody that specifically binds to CD47 comprises CDR sequences selected from those comprised in the following heavy chain variable regions and light chain variable regions:

    (1) an HCDR1, an HCDR2 and an HCDR3 comprised in a heavy chain variable region set forth in SEQ ID NO: 2, and
    an LCDR1, an LCDR2 and an LCDR3 comprised in a light chain variable region set forth in SEQ ID NO: 4; or
    (2) an HCDR1, an HCDR2 and an HCDR3 comprised in a heavy chain variable region set forth in SEQ ID NO: 12, and
    an LCDR1, an LCDR2 and an LCDR3 comprised in a light chain variable region set forth in SEQ ID NO: 14; or
    (3) an HCDR1, an HCDR2 and an HCDR3 comprised in a heavy chain variable region set forth in SEQ ID NO: 16, and
    an LCDR1, an LCDR2 and an LCDR3 comprised in a light chain variable region set forth in SEQ ID NO: 18; or
    (4) an HCDR1, an HCDR2 and an HCDR3 comprised in a heavy chain variable region set forth in SEQ ID NO: 20, and

    an LCDR1, an LCDR2 and an LCDR3 comprised in a light chain variable region set forth in SEQ ID NO: 22 (preferably, according to the IMGT numbering system, the antibody comprises:

    an HCDR1 comprising or consisting of a sequence set forth in SEQ ID NO: 5 or a variant thereof, an HCDR2 comprising or consisting of a sequence set forth in SEQ ID NO: 6 or a variant thereof, and an HCDR3 comprising or consisting of a sequence set forth in SEQ ID NO: 7 or a variant thereof; and the antibody further comprises:

    an LCDR1 comprising or consisting of amino acids set forth in SEQ ID NO: 8 or a variant thereof, an LCDR2 comprising or consisting of an amino acid sequence set forth in SEQ ID NO: 9 or a variant thereof, and an LCDR3 comprising or consisting of a sequence set forth in SEQ ID NO: 10 or a variant thereof);
    the bispecific antibody is an anti-PD-1/anti-CTLA4 antibody or an anti-PD-1/anti-VEGFA antibody or a combination of the two; the bispecific antibody comprises a first protein functional region and a second protein functional region, wherein the first protein functional region targets PD-1, and the second protein functional region targets CTLA4 or VEGFA; the first protein functional region is an immunoglobulin, and the second protein functional region is a single-chain antibody; or the first protein functional region is a single-chain antibody, and the second protein functional region is an immunoglobulin; preferably, two single-chain antibody molecules (preferably, two identical single-chain antibody molecules) are linked to one immunoglobulin molecule; preferably, the immunoglobulin is of IgG1 subtype (preferably, human IgG1 subtype); preferably, the single-chain antibody is linked to the N terminus or C terminus of the heavy chain of the immunoglobulin, wherein the numbers of the first protein functional region and the second protein functional region are each independently 1, 2 or more; preferably, the first protein functional region is linked to the second protein functional region either directly or via a first linker fragment; and/or the heavy chain variable region of the single-chain antibody is linked to the light chain variable region of the single-chain antibody either directly or via a second linker fragment; the first linker fragment and the second linker fragment are identical or different; preferably, the linker fragment is (GGGGS)n, wherein n is a positive integer, or more preferably, n is 1, 2, 3, 4, 5 or 6; preferably, the first linker fragment and the second linker fragment comprise amino acid sequences independently selected from SEQ ID NO: 119 and SEQ ID NO: 120; more preferably, the first linker fragment and the second linker fragment comprise an amino acid sequence set forth in SEQ ID NO: 120;

when the bispecific antibody is an anti-PD-1/anti-CTLA4 antibody,

the first protein functional region comprises HCDR1-HCDR3 comprised in a heavy chain variable region set forth in SEQ ID NO: 87, and LCDR1-LCDR3 comprised in a light chain variable region set forth in SEQ ID NO: 88 (preferably, according to the IMGT numbering system, the first protein functional region comprises an HCDR1 comprising or consisting of a sequence set forth in SEQ ID NO: 89 or a variant thereof,

an HCDR2 comprising or consisting of a sequence set forth in SEQ ID NO: 90 or a variant thereof,

an HCDR3 comprising or consisting of a sequence set forth in SEQ ID NO: 91 or a variant thereof,

an LCDR1 comprising or consisting of amino acids set forth in SEQ ID NO: 92 or a variant thereof,

an LCDR2 comprising or consisting of an amino acid sequence set forth in SEQ ID NO: 93 or a variant thereof, and

an LCDR3 comprising or consisting of a sequence set forth in SEQ ID NO: 94 or a variant thereof);

the second protein functional region comprises HCDR1-HCDR3 in a heavy chain variable region set forth in SEQ ID NO: 95, and LCDR1-LCDR3 in a light chain variable region set forth in SEQ ID NO: 96 (preferably, according to the IMGT numbering system, the second protein functional region comprises an HCDR1 comprising or consisting of a sequence set forth in SEQ ID NO: 97 or a variant thereof,

an HCDR2 comprising or consisting of a sequence set forth in SEQ ID NO: 98 or a variant thereof,

an HCDR3 comprising or consisting of a sequence set forth in SEQ ID NO: 99 or a variant thereof,

an LCDR1 comprising or consisting of amino acids set forth in SEQ ID NO: 100 or a variant thereof,

an LCDR2 comprising or consisting of an amino acid sequence set forth in SEQ ID NO: 101 or a variant thereof, and

an LCDR3 comprising or consisting of a sequence set forth in SEQ ID NO: 102 or a variant thereof);

or

when the bispecific antibody is an anti-PD-1/anti-VEGFA antibody,

the first protein functional region comprises HCDR1-HCDR3 comprised in a heavy chain variable region set forth in SEQ ID NO: 103, and LCDR1-LCDR3 comprised in a light chain variable region set forth in SEQ ID NO: 104 (preferably, according to the IMGT numbering system, the first protein functional region comprises an HCDR1 comprising or consisting of a sequence set forth in SEQ ID NO: 105 or a variant thereof,

an HCDR2 comprising or consisting of a sequence set forth in SEQ ID NO: 106 or a variant thereof,

an HCDR3 comprising or consisting of a sequence set forth in SEQ ID NO: 107 or a variant thereof,

an LCDR1 comprising or consisting of amino acids set forth in SEQ ID NO: 108 or a variant thereof,

an LCDR2 comprising or consisting of an amino acid sequence set forth in SEQ ID NO: 109 or a variant thereof, and

an LCDR3 comprising or consisting of a sequence set forth in SEQ ID NO: 110 or a variant thereof);

the second protein functional region comprises HCDR1-HCDR3 in a heavy chain variable region set forth in SEQ ID NO: 111, and LCDR1-LCDR3 in a light chain variable region set forth in SEQ ID NO: 112 (preferably, according to the IMGT numbering system, the second protein functional region comprises an HCDR1 comprising or consisting of a sequence set forth in SEQ ID NO: 113 or a variant thereof,

an HCDR2 comprising or consisting of a sequence set forth in SEQ ID NO: 114 or a variant thereof,

an HCDR3 comprising or consisting of a sequence set forth in SEQ ID NO: 115 or a variant thereof,

an LCDR1 comprising or consisting of amino acids set forth in SEQ ID NO: 116 or a variant thereof,

an LCDR2 comprising or consisting of an amino acid sequence set forth in SEQ ID NO: 117 or a variant thereof, and

an LCDR3 comprising or consisting of a sequence set forth in SEQ ID NO: 118 or a variant thereof);

wherein,

the anti-PD1 monoclonal antibody comprises HCDR1-HCDR3 comprised in a heavy chain variable region set forth in SEQ ID NO: 121, and LCDR1-LCDR3 comprised in a light chain variable region set forth in SEQ ID NO: 122 (preferably, according to the IMGT numbering system, the anti-PD1 monoclonal antibody comprises an HCDR1 comprising or consisting of a sequence set forth in SEQ ID NO: 123 or a variant thereof,

an HCDR2 comprising or consisting of a sequence set forth in SEQ ID NO: 124 or a variant thereof,

an HCDR3 comprising or consisting of a sequence set forth in SEQ ID NO: 125 or a variant thereof,

an LCDR1 comprising or consisting of amino acids set forth in SEQ ID NO: 126 or a variant thereof,

an LCDR2 comprising or consisting of an amino acid sequence set forth in SEQ ID NO: 127 or a variant thereof, and

an LCDR3 comprising or consisting of a sequence set forth in SEQ ID NO: 128 or a variant thereof);

wherein the variant comprises a sequence having at least 80%, 81%, 82%, 83%, 84%, 85%, 86%, 87%, 88%, 89%, 90%, 91%, 92%, 93%, 94%, 95%, 96%, 97%, 98%, 99% homology to the corresponding sequence, or the variant comprises a sequence having one or more (preferably 1, 2 or 3) conservative amino acid mutations (preferably substitutions, insertions or deletions) compared to the corresponding sequence.

2. The pharmaceutical composition according to claim 1, wherein the antibody that specifically binds to CD47 further comprises framework regions (FRs) in the heavy chain variable region and framework regions (FRs) in the light chain variable region selected from the group consisting of the following:

(1) the framework regions (FRs) in the heavy chain variable region including an FR-H1, an FR-H2, an FR-H3 and an FR-H4, wherein the FR-H1 comprises or consists of an amino acid sequence set forth in SEQ ID NO: 23 or a variant thereof; the FR-H2 comprises or consists of an amino acid sequence set forth in SEQ ID NO: 24 or a variant thereof; the FR-H3 comprises or consists of an amino acid sequence set forth in SEQ ID NO: 25 or a variant thereof; the FR-H4 comprises or consists of an amino acid sequence set forth in SEQ ID NO: 26 or a variant thereof; the framework regions (FRs) in the light chain variable region including an FR-L1, an FR-L2, an FR-L3 and an FR-L4, wherein the FR-L1 comprises or consists of an amino acid sequence set forth in SEQ ID NO: 27 or a variant thereof; the FR-L2 comprises or consists of an amino acid sequence set forth in SEQ ID NO: 28 or a variant thereof; the FR-L3 comprises or consists of an amino acid sequence set forth in SEQ ID NO: 29 or a variant thereof; the FR-L4 comprises or consists of an amino acid sequence set forth in SEQ ID NO: 30 or a variant thereof;

(2) the framework regions (FRs) in the heavy chain variable region including an FR-H1, an FR-H2, an FR-H3 and an FR-H4, wherein the FR-H1 comprises or consists of an amino acid sequence set forth in SEQ ID NO: 31 or a variant thereof; the FR-H2 comprises or consists of an amino acid sequence set forth in SEQ ID NO: 32 or a variant thereof; the FR-H3 comprises or consists of an amino acid sequence set forth in SEQ ID NO: 33 or a variant thereof; the FR-H4 comprises or consists of an amino acid sequence set forth in SEQ ID NO: 34 or a variant thereof; the framework regions (FRs) in the light chain variable region including an FR-L1, an FR-L2, an FR-L3 and an FR-L4, wherein the FR-L1 comprises or consists of an amino acid sequence set forth in SEQ ID NO: 35 or a variant thereof; the FR-L2 comprises or consists of an amino acid sequence set forth in SEQ ID NO: 36 or a variant thereof; the FR-L3 comprises or consists of an amino acid sequence set forth in SEQ ID NO: 37 or a variant thereof; the FR-L4 comprises or consists of an amino acid sequence set forth in SEQ ID NO: 38 or a variant thereof;

(3) the framework regions (FRs) in the heavy chain variable region including an FR-H1, an FR-H2, an FR-H3 and an FR-H4, wherein the FR-H1 comprises or consists of an amino acid sequence set forth in SEQ ID NO:

39 or a variant thereof; the FR-H2 comprises or consists of an amino acid sequence set forth in SEQ ID NO: 40 or a variant thereof; the FR-H3 comprises or consists of an amino acid sequence set forth in SEQ ID NO: 41 or a variant thereof; the FR-H4 comprises or consists of an amino acid sequence set forth in SEQ ID NO: 42 or a variant thereof; the framework regions (FRs) in the light chain variable region including an FR-L1, an FR-L2, an FR-L3 and an FR-L4, wherein the FR-L1 comprises or consists of an amino acid sequence set forth in SEQ ID NO: 43 or a variant thereof; the FR-L2 comprises or consists of an amino acid sequence set forth in SEQ ID NO: 44 or a variant thereof; the FR-L3 comprises or consists of an amino acid sequence set forth in SEQ ID NO: 45 or a variant thereof; the FR-L4 comprises or consists of an amino acid sequence set forth in SEQ ID NO: 46 or a variant thereof;

(4) the framework regions (FRs) in the heavy chain variable region including an FR-H1, an FR-H2, an FR-H3 and an FR-H4, wherein the FR-H1 comprises or consists of an amino acid sequence set forth in SEQ ID NO: 47 or a variant thereof; the FR-H2 comprises or consists of an amino acid sequence set forth in SEQ ID NO: 48 or a variant thereof; the FR-H3 comprises or consists of an amino acid sequence set forth in SEQ ID NO: 49 or a variant thereof; the FR-H4 comprises or consists of an amino acid sequence set forth in SEQ ID NO: 50 or a variant thereof; the framework regions (FRs) in the light chain variable region including an FR-L1, an FR-L2, an FR-L3 and an FR-L4, wherein the FR-L1 comprises or consists of an amino acid sequence set forth in SEQ ID NO: 51 or a variant thereof; the FR-L2 comprises or consists of an amino acid sequence set forth in SEQ ID NO: 52 or a variant thereof; the FR-L3 comprises or consists of an amino acid sequence set forth in SEQ ID NO: 53 or a variant thereof; the FR-L4 comprises or consists of an amino acid sequence set forth in SEQ ID NO: 54 or a variant thereof,
wherein the variant comprises a sequence having at least 80%, preferably at least 81%, 82%, 83%, 84%, 85%, 86%, 87%, 88%, 89%, 90%, 91%, 92%, 93%, 94%, 95%, 96%, 97%, 98% or 99% homology to the corresponding sequence or having one or more (preferably 1, 2, 3, 4, 5, 6, 7, 8, 9 or 10) conservative amino acid mutations (preferably substitutions, insertions or deletions) compared to the corresponding sequence.

3. The pharmaceutical composition according to claim 1 or 2, wherein the antibody that specifically binds to CD47 comprises:

(1) a heavy chain variable region comprising or consisting of:

an amino acid sequence set forth in SEQ ID NO: 2 or a variant thereof, and
a light chain variable region comprising or consisting of:
an amino acid sequence set forth in SEQ ID NO: 4 or a variant thereof;

(2) a heavy chain variable region comprising or consisting of:

an amino acid sequence set forth in SEQ ID NO: 12 or a variant thereof, and
a light chain variable region comprising or consisting of:
an amino acid sequence set forth in SEQ ID NO: 14 or a variant thereof;

(3) a heavy chain variable region comprising or consisting of:

an amino acid sequence set forth in SEQ ID NO: 16 or a variant thereof, and
a light chain variable region comprising or consisting of:
an amino acid sequence set forth in SEQ ID NO: 18 or a variant thereof; and

(4) a heavy chain variable region comprising or consisting of:

an amino acid sequence set forth in SEQ ID NO: 20 or a variant thereof, and
a light chain variable region comprising or consisting of:

an amino acid sequence set forth in SEQ ID NO: 22 or a variant thereof,
the first protein functional region of the anti-PD-1/anti-CTLA4 antibody comprises:

(1) a heavy chain variable region comprising or consisting of:

a sequence set forth in SEQ ID NO: 87 or a variant thereof, and
a light chain variable region comprising or consisting of:

a sequence set forth in SEQ ID NO: 88 or a variant thereof; and

(2) a heavy chain variable region comprising or consisting of:

a sequence set forth in SEQ ID NO: 129 or a variant thereof, and
a light chain variable region comprising or consisting of:

a sequence set forth in SEQ ID NO: 130 or a variant thereof;
the second protein functional region of the anti-PD-1/anti-CTLA4 antibody comprises:

(1) a heavy chain variable region comprising or consisting of:

a sequence set forth in SEQ ID NO: 95 or a variant thereof, and
a light chain variable region comprising or consisting of:
a sequence set forth in SEQ ID NO: 96 or a variant thereof;

(2) a heavy chain variable region comprising or consisting of:

a sequence set forth in SEQ ID NO: 133 or a variant thereof, and
a light chain variable region comprising or consisting of:
a sequence set forth in SEQ ID NO: 134 or a variant thereof;

(3) a heavy chain variable region comprising or consisting of:

a sequence set forth in SEQ ID NO: 135 or a variant thereof, and
a light chain variable region comprising or consisting of:
a sequence set forth in SEQ ID NO: 136 or a variant thereof;

(4) a heavy chain variable region comprising or consisting of:

a sequence set forth in SEQ ID NO: 137 or a variant thereof, and
a light chain variable region comprising or consisting of:
a sequence set forth in SEQ ID NO: 138 or a variant thereof; and

(5) a heavy chain variable region comprising or consisting of:

a sequence set forth in SEQ ID NO: 131 or a variant thereof, and
a light chain variable region comprising or consisting of:

a sequence set forth in SEQ ID NO: 132 or a variant thereof;
preferably, the immunoglobulin of the anti-PD-1/anti-CTLA4 antibody comprises a heavy chain having an amino acid sequence set forth in SEQ ID NO: 139, and targets PD-1;
the first protein functional region of the anti-PD-1/anti-VEGFA antibody comprises:

(1) a heavy chain variable region comprising or consisting of:

a sequence set forth in SEQ ID NO: 103 or a variant thereof, and
a light chain variable region comprising or consisting of:

a sequence set forth in SEQ ID NO: 104 or a variant thereof;
the second protein functional region of the anti-PD-1/anti-VEGFA antibody comprises:

(1) a heavy chain variable region comprising or consisting of:

a sequence set forth in SEQ ID NO: 111 (preferably encoded by a nucleotide sequence set forth in SEQ ID NO: 145) or a variant thereof, and
a light chain variable region comprising or consisting of:

a sequence set forth in SEQ ID NO: 112 (preferably encoded by a nucleotide sequence set forth in SEQ ID NO: 146) or a variant thereof;
preferably, the immunoglobulin of the anti-PD-1/anti-VEGFA antibody comprises a heavy chain having an amino acid sequence set forth in SEQ ID NO: 140, and targets VEGFA;
the anti-PD-1 monoclonal antibody comprises or consists of a heavy chain variable region set forth in SEQ ID NO: 121 and a light chain variable region set forth in SEQ ID NO: 122;
wherein the variant has at least 85%, preferably 86%, 87%, 88%, 89%, 90%, 91%, 92%, 93%, 94%, 95%, 96%, 97%, 98% or 99% or higher sequence identity to the corresponding sequence, or comprises an amino acid sequence having one or more (preferably 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30) conservative amino acid mutations (preferably substitutions, insertions or deletions) compared to the corresponding sequence.

4. The pharmaceutical composition according to any one of claims 1-3, wherein the antibody that specifically binds to CD47 further comprises a heavy chain constant region and a light chain constant region, and the constant regions are derived from species other than murine, e.g., from a human antibody, preferably from a human IgG or IgM, and more preferably from IgG1; preferably, the heavy chain constant region is Ig gamma-1 chain C region, ACCESSION No. P01857 (SEQ ID NO: 58) or Ig gamma-4 chain C region, ACCESSION No. P01861.1 (SEQ ID NO: 56); the light chain constant region is Ig kappa chain C region, ACCESSION No. P01834 (SEQ ID NO: 57); preferably, the antibody that specifically binds to CD47 is secreted by a hybridoma cell line LT012 under CCTCC NO. 2018135.

5. The pharmaceutical composition according to any one of claims 1-4, wherein, according to the EU numbering system, the antibody that specifically binds to CD47 and the immunoglobulin comprise a heavy chain constant region having mutations at any 1, 2 or 3 of positions 234, 235 and 237, and the affinity constant of the bispecific antibody for FcγRIIIa and/or C1q is reduced after the mutation as compared to that before the mutation; preferably, the affinity constant is measured by a Fortebio Octet system;

preferably, the antibody that specifically binds to CD47 comprises an L234A and/or L235A mutation according to the EU numbering system;
more preferably, the heavy chain constant region of the immunoglobulin has one of the following combinations of mutations:

L234A and L235A; or
L234A and G237A; or
L235A and G237A; or
L234A, L235A and G237A;
still more preferably,
according to the EU numbering system, the heavy chain constant region of the immunoglobulin further has one of the combinations of the following mutations:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

6. The pharmaceutical composition according to any one of claims 1-5, wherein the antibody that specifically binds to CD47 comprises or consists of a heavy chain and a light chain selected from the group consisting of the following:

(1) a heavy chain set forth in SEQ ID NO: 59 and a light chain set forth in SEQ ID NO: 60;
(2) a heavy chain set forth in SEQ ID NO: 61 and a light chain set forth in SEQ ID NO: 62;
(3) a heavy chain set forth in SEQ ID NO: 63 and a light chain set forth in SEQ ID NO: 64;
(4) a heavy chain set forth in SEQ ID NO: 65 and a light chain set forth in SEQ ID NO: 66;
(5) a heavy chain set forth in SEQ ID NO: 67 and a light chain set forth in SEQ ID NO: 68; and
(6) a heavy chain set forth in SEQ ID NO: 69 and a light chain set forth in SEQ ID NO: 70;

the anti-PD-1/anti-CTLA4 antibody comprises or consists of a heavy chain and a light chain selected from the group consisting of the following:
a heavy chain set forth in SEQ ID NO: 78 (preferably encoded by a nucleotide sequence set forth in SEQ ID NO: 77) and a light chain set forth in SEQ ID NO: 80 (preferably encoded by a nucleotide sequence set forth in SEQ ID NO: 79);
the anti-PD-1/anti-VEGFA antibody comprises or consists of a heavy chain and a light chain selected from the group consisting of the following:
a heavy chain set forth in SEQ ID NO: 141 (preferably encoded by a nucleotide sequence set forth in SEQ ID NO: 142) and a light chain set forth in SEQ ID NO: 143 (preferably encoded by a nucleotide sequence set forth in SEQ ID NO: 144); the anti-PD-1 monoclonal antibody comprises or consists of a heavy chain and a light chain selected from the group consisting of the following: a heavy chain set forth in SEQ ID NO: 82 (preferably encoded by a nucleotide sequence set forth in SEQ ID NO: 81) and a light chain set forth in SEQ ID NO: 84 (preferably encoded by a nucleotide sequence set forth in SEQ ID NO: 83).

7. The pharmaceutical composition according to any one of claims 1-6, wherein the antigen-binding fragment is selected from Fab, Fab', F(ab')$_2$, Fd, Fv, dAb, Fab/c, complementarity determining region (CDR) fragment, single-chain antibody (e.g., scFv), bivalent antibody and domain antibody.

8. The pharmaceutical composition according to any one of claims 1-7, wherein the antibody that specifically binds to CD47 is a humanized antibody, a chimeric antibody or a multispecific antibody (e.g., a bispecific antibody).

9. The pharmaceutical composition according to any one of claims 1-8, wherein the antibody that specifically binds to CD47 binds to human CD47 protein with a KD less than about $10^{-5}$ M, e.g., less than about $10^{-6}$ M, $10^{-7}$ M, $10^{-8}$ M, $10^{-9}$ M, or $10^{-10}$ M or less, or the antibody that specifically binds to CD47 binds to human CD47 protein with an $EC_{50}$ less than about 100 nM, e.g., less than about 10 nM, 1 nM, 0.9 nM, 0.8 nM, 0.7 nM, 0.6 nM, 0.5 nM, 0.4 nM, 0.3 nM, 0.2 nM, 0.1 nM or less.

10. The pharmaceutical composition according to any one of claims 1-9, wherein the antibody that specifically binds to CD47 is in the form of an antibody conjugate comprising the antibody that specifically binds to CD47 or the antigen-binding fragment thereof according to any one of claims 1-9 and a conjugated moiety conjugated thereto, wherein the conjugated moiety is a purification tag (e.g., a His tag), a cytotoxic agent or a detectable label; preferably, the conjugated moiety is a radioisotope, a luminescent substance, a colored substance, an enzyme or polyethylene glycol; or
the antibody that specifically binds to CD47 is in the form of a multispecific antibody, preferably a bispecific antibody, comprising the antibody that specifically binds to CD47 or the antigen-binding fragment thereof according to any one of claims 1-9, and an antibody against another antigen and/or another antigenic epitope or an antigen-binding fragment thereof; or the antibody that specifically binds to CD47 is in the form of a fusion protein comprising the antibody that specifically binds to CD47 or the antigen-binding fragment thereof according to any one of claims 1-9.

11. The pharmaceutical composition according to any one of claims 1-10, wherein the anti-tumor therapeutic agent is selected from one or more of the following: a tyrosine kinase inhibitor, a DNA polymerase inhibitor, an anti-human CD20 antibody, an anti-human PDL-1 antibody, an anti-human PD-1 antibody, an anti-human CTLA-4 antibody, a BCL-2 inhibitor, an anti-human EGFR antibody, an anti-human HER2 antibody, an anti-human HER3 antibody, acalabrutinib, a cyclin-dependent kinase inhibitor, an anti-human VEGFR2 antibody, an anti-human VEGF antibody, a proteasome inhibitor, an angiogenesis inhibitor, a rapidly accelerated fibrosarcoma (RAF) inhibitor, a bispecific antibody and a fusion protein drug; preferably, the therapeutic agent is cetuximab, obinutuzumab or rituximab; preferably, the anti-tumor chemotherapeutic is selected from one or more of the following: an alkylating agent, an anthracycline, an antimetabolite, an antibiotic, a plant-based and/or hormonal drug, a platinum-based drug (e.g., cisplatin, carboplatin and oxaliplatin), adriamycin, cyclophosphamide, a taxane (e.g., albumin-bound paclitaxel, liposome paclitaxel and docetaxel), etoposide, gemcitabine, pemetrexed, capecitabine, olaparib, rucaparib, niraparib, talazoparib, fluzoparib, vinca alkaloid, tamoxifen, megestrol, goserelin, asparaginase, a fluorouracil antineoplastic drug (e.g., 5-fluorouracil), azacitidine, cytarabine and cyclocytidine.

12. The pharmaceutical composition according to any one of claims 1-11, wherein the pharmaceutical composition further comprises a pharmaceutically acceptable carrier and/or excipient.

13. The pharmaceutical composition according to any one of claims 1-12, wherein, based on the mass of the antibody, the component A and the component B1 are in a mass ratio of (1:5)-(5:1), e.g., 1:5, 1:4, 1:3, 1:2, 1:1, 2:1, 3:1, 4:1 or 5:1;

the component A and the component B3 are in a mass ratio of (1:5)-(5:1), e.g., 1:5, 1:4, 1:3, 1:2, 1:1, 2:1, 3:1, 4:1 or 5:1;
or
the component B2 and the component A are in a mass ratio of 1:(1-1000), preferably 1:(5-500), and more preferably 1:(10-100);
preferably, the component A and the component B in the pharmaceutical composition are in a form suitable for administration by intravenous drip infusion, subcutaneous injection, intradermal injection, intravenous injection, intramuscular injection or intralesional injection.

14. A kit, preferably for treating a tumor, comprising a product A and a product B, wherein the product A comprises the antibody that specifically binds to CD47 or the antigen-binding fragment thereof as defined in any one of claims 1-13;

the product B is selected from one or more of the following: a product B1, a product B2 and a product B3, wherein the product B1 is the bispecific antibody or the antigen-binding fragment thereof as defined in any one of claims 1-13 (preferably, when the bispecific antibody is a combination of an anti-PD-1/anti-CTLA4 antibody and an anti-PD-1/anti-VEGFA antibody, the anti-PD-1/anti-CTLA4 antibody and the anti-PD-1/anti-VEGFA antibody are packaged either separately or together), the product B2 is the anti-tumor chemotherapeutic as defined in any one of claims 1-13, and the product B3 is the anti-PD-1 monoclonal antibody or the antigen-binding fragment thereof as defined in any one of claims 1-13, wherein the product B1, the product B2 and the product B3 are packaged either separately or together; preferably, the kit further comprises a product C, wherein the product C is the anti-tumor therapeutic agent as defined in any one of claims 1-13, and the product C is different from the product B;
preferably, the kit further comprises a package insert.

15. The kit according to claim 14, wherein, based on the mass of the antibody, the product A and the product B1 are in a mass ratio of (1:5)-(5:1), e.g., 1:5, 1:4, 1:3, 1:2, 1:1,2:1,3:1,4:1 or 5:1;

the product A and the product B3 are in a mass ratio of (1:5)-(5:1), e.g., 1:5, 1:4, 1:3, 1:2, 1:1, 2:1, 3:1, 4:1 or 5:1;
or
the anti-tumor chemotherapeutic is in a unit dose of 0.1-100 mg, 0.5-50 mg, 1-20 mg, 2-15 mg, 4-12 mg or 8-12 mg;
preferably, the product A, the product B or the product C in the kit is in a form suitable for administration by intravenous drip infusion, subcutaneous injection, intradermal injection, intravenous injection, intramuscular injection or intralesional injection.

16. A method for treating or preventing a tumor, comprising administering to a subject in need an effective amount of the component A and the component B as defined in any one of claims 1-13, wherein preferably, the method further comprises administering to the subject in need an effective amount of the component C as defined in any one of claims 1-13;

preferably, the component A, the component B and the component C are administered simultaneously or sequentially; more preferably, the component A, the component B and the component C are administered before or after a surgical treatment and/or before or after a radiotherapy;
preferably, when the component B is the component B1 and/or the component B3, the component A or the component B is administered at a unit dose of 0.1-100 mg, preferably 1-10 mg per kg body weight of the subject; or the component A or the component B is administered at a unit dose of 10-1000 mg, preferably 50-500 mg, 100-400 mg, 150-300 mg, 150-250 mg or 200 mg in each subject;
preferably, the component B2 is in a unit dose of 0.1-100 mg, 0.5-50 mg, 1-20 mg, 2-15 mg, 4-12 mg or 8-12 mg;

preferably, the dose is administered from twice daily to about once every other day, or once every 3 days, 4 days, 5 days, 6 days, 10 days, 1 week, 2 weeks, 3 weeks, 4 weeks, 5 weeks or 6 weeks;
preferably, the route of administration is intravenous drip infusion, subcutaneous injection, intradermal injection, intravenous injection, intramuscular injection or intralesional injection.

17. The pharmaceutical composition according to any one of claims 1-13, the kit according to claim 14 or 15, or the method according to claim 16, wherein the tumor is preferably a tumor expressing CD47, and preferably a cancer; the cancer includes a solid tumor, a hematological tumor, lymphoma, blastoma, sarcoma, leukemia or lymphoid malignancy, and more preferably includes squamous cell carcinoma, myeloma, lung cancer, small cell lung cancer, non-small cell lung cancer, head and neck squamous cell carcinoma, glioma (e.g., neuroglioma and recurrent glioma), acute myelocytic leukemia, Hodgkin's lymphoma, non-Hodgkin's lymphoma, diffuse large B-cell lymphoma, follicular lymphoma, acute lymphocytic leukemia, acute myeloblastic leukemia, chronic lymphocytic leukemia, chronic myeloblastic leukemia, primary mediastinal large B-cell lymphoma, mantle cell lymphoma, small lymphocytic lymphoma, T cell/histiocyte-rich large B-cell lymphoma, multiple myeloma, myeloid leukemia-1 protein, relapsed and refractory peripheral T-cell lymphoma, myelodysplastic syndrome, anaplastic large cell lymphoma, mantle cell lymphoma, marginal zone lymphoma, myelofibrosis, polycythemia vera, bone marrow cancer, myeloproliferative disease, aggressive systemic mastocytosis, eosinophilia, dermatofibrosarcoma protuberans, chronic eosinophilic leukemia, gastrointestinal cancer, gastric adenocarcinoma or gastroesophageal junction adenocarcinoma, ovarian cancer, liver cancer, lymphocytic leukemia, large intestine cancer, endometrial cancer, prostate cancer, thyroid cancer, melanoma, chondrosarcoma, neuroblastoma, adenocarcinoma, pancreatic cancer, pancreatic ductal adenocarcinoma, glioblastoma multiforme, bone cancer, Ewing's sarcoma, cervical cancer, nasopharyngeal cancer, brain cancer, bladder cancer, breast cancer, triple negative breast cancer, intestinal cancer, rectal cancer, colorectal cancer, colon cancer, hepatocellular carcinoma, renal cell carcinoma, clear cell renal cell carcinoma, head and neck cancer, throat cancer, hepatobiliary cancer, central nervous system cancer, esophageal carcinoma, esophageal squamous cell carcinoma, malignant pleural mesothelioma, systemic light chain amyloidosis, lymphocytic lymphoma, myeloproliferative neoplasm, neuroendocrine tumor, Merkel cell carcinoma, testicular cancer and skin cancer, peritoneal cancer, fallopian tube cancer, urothelial cancer, microsatellite instability-high (MSI-H) or mismatch repair deficient (dMMR) cancer and mesothelioma.

18. A unit formulation, preferably for treating a tumor, comprising 1-10000 mg (preferably 10-1000 mg, and preferably 50-500 mg, 100-400 mg, 150-300 mg, 150-250 mg or 200 mg) of the component A and the component B as defined in any one of claims 1-13,

wherein, when the component B is the component B1 and/or the component B3, the unit formulation comprises 1-10000 mg (preferably 1-1000 mg, and preferably 50-500 mg, 100-400 mg, 150-300 mg, 150-250 mg, 200 mg or 100 mg) of the component B as defined in any one of claims 1-13;
when the component B is the component B2, the unit formulation comprises 0.1-100 mg, 0.5-50 mg, 1-20 mg, 2-15 mg, 4-12 mg, or 8-12 mg of the component B; preferably, the unit formulation further comprises one or more of the components C as defined in any one of claims 1-13;
wherein the component A, the component B and the component C are packaged separately.

19. A single dose unit, preferably for treating a tumor, comprising 0.1-10000 mg (preferably 1-1000 mg, and preferably 50-500 mg, 100-400 mg, 150-300 mg, 150-250 mg, 200 mg or 100 mg) of the component A and the component B as defined in any one of claims 1-13,

wherein, when the component B is the component B1 and/or the component B3, the single dose unit comprises 0.1-10000 mg (preferably 1-1000 mg, and preferably 50-500 mg, 100-400 mg, 150-300 mg, 150-250 mg, 200 mg or 100 mg) of the component B as defined in any one of claims 1-13;
when the component B is the component B2, the single dose unit comprises 0.1-100 mg, 0.5-50 mg, 1-20 mg, 2-15 mg, 4-12 mg, or 8-12 mg of the component B.

FIG. 1

FIG. 2

FIG. 3

FIG. 4

FIG. 5

FIG. 6

FIG. 7

FIG. 8

FIG. 9

FIG. 10

FIG. 11

FIG. 12

## INTERNATIONAL SEARCH REPORT

| International application No. |
| --- |
| **PCT/CN2022/086890** |

**A. CLASSIFICATION OF SUBJECT MATTER**

C07K 16/28(2006.01)i; C12N 15/13(2006.01)i; C07K 19/00(2006.01)i

According to International Patent Classification (IPC) or to both national classification and IPC

**B. FIELDS SEARCHED**

Minimum documentation searched (classification system followed by classification symbols)

C07K; C12N

Documentation searched other than minimum documentation to the extent that such documents are included in the fields searched

Electronic data base consulted during the international search (name of data base and, where practicable, search terms used)

CNTXT, ENTXT, VEN, ISI, 中国专利生物序列检索系统, China Patents Biological Sequence Search System, NCBI, 百度学术, BAIDU SCHOLAR, CNKI, 万方数据库, WANFANG DATABASE, PUBMED: 发明人, 康方生物, CD47, IAP, 整合素相关蛋白, integrin associated protein, azacitidine, 阿扎胞苷, 利妥昔单抗, rituximabs, LT012, penpulimab, cadonilimab, VP101, PD-1, antibody, 化疗药, 序列检索, sequence search

**C. DOCUMENTS CONSIDERED TO BE RELEVANT**

| Category* | Citation of document, with indication, where appropriate, of the relevant passages | Relevant to claim No. |
| --- | --- | --- |
| Y | CN 112442123 A (AKESO BIOPHARMA, INC.) 05 March 2021 (2021-03-05) claim 25, and description, paragraphs 10-29 and 64-85 | 1-19 |
| Y | CN 112300286 A (AKESO PHARMACEUTICALS INC.) 02 February 2021 (2021-02-02) abstract, claims 1-28, and description, paragraphs 329-403 | 1-19 |
| PY | CN 112830972 A (AKESO BIOPHARMA, INC.) 25 May 2021 (2021-05-25) claims 1-22 | 1-19 |
| X | WO 2021004480 A1 (GENSCRIPT (NANJING) CO., LTD.) 14 January 2021 (2021-01-14) abstract, and pages 9-10 | 1, 7-19 |
| Y | WO 2021004480 A1 (GENSCRIPT (NANJING) CO., LTD.) 14 January 2021 (2021-01-14) abstract, and pages 9-10 | 1-19 |
| X | FENG, Dongdong et al. "Combination treatment with 5F9 and azacitidine enhances phagocytic elimination of acute myeloid leukemia" *Blood,* Vol. 132, No. supplement 1, 29 November 2018 (2018-11-29), abstract | 1, 7-19 |

☑ Further documents are listed in the continuation of Box C.    ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **01 July 2022** | **11 July 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/086890** |

**C.      DOCUMENTS CONSIDERED TO BE RELEVANT**

| Category* | Citation of document, with indication, where appropriate, of the relevant passages | Relevant to claim No. |
| --- | --- | --- |
| Y | FENG, Dongdong et al. "Combination treatment with 5F9 and azacitidine enhances phagocytic elimination of acute myeloid leukemia" *Blood*, Vol. 132, No. supplement 1, 29 November 2018 (2018-11-29), abstract | 1-19 |
| X | SHU, Lian et al. "Dual blockage of both PD-L1 and CD47 enhances immunotherapy against circulating tumor cells" *SCIENTIFIC REPORTS*, Vol. 9, 14 March 2019 (2019-03-14), 4532 abstract, figure 4, and page 6, paragraph 1 | 1, 7-19 |
| Y | SHU, Lian et al. "Dual blockage of both PD-L1 and CD47 enhances immunotherapy against circulating tumor cells" *SCIENTIFIC REPORTS*, Vol. 9, 14 March 2019 (2019-03-14), 4532 abstract, figure 4, and page 6, paragraph 1 | 1-19 |
| X | HUA, Tao et al. "Targeting CD47 enhances the efficacy of anti-PD-1 and CTLA-4 in an esophageal squamous cell cancer preclinical model." *ONCOLOGY RESEARCH*, Vol. 25, No. 9, 23 March 2017 (2017-03-23), abstract, and page 1580, left column, and figure 4 | 1, 7-19 |
| Y | HUA, Tao et al. "Targeting CD47 enhances the efficacy of anti-PD-1 and CTLA-4 in an esophageal squamous cell cancer preclinical model." *ONCOLOGY RESEARCH*, Vol. 25, No. 9, 23 March 2017 (2017-03-23), abstract, and page 1580, left column, and figure 4 | 1-19 |
| X | ZHAO, Lei et al. "Computational design of novel tetra-specific antibody (PD-1/CD47/VEGF/ TGF-$\beta$) with IgG-like architecture against non-small cell lung cancer (NSCLC)." *JOURNAL OF CLINICAL ONCOLOGY*, Vol. 37, 26 May 2019 (2019-05-26), abstract | 1, 7-19 |
| Y | ZHAO, Lei et al. "Computational design of novel tetra-specific antibody (PD-1/CD47/VEGF/ TGF-$\beta$) with IgG-like architecture against non-small cell lung cancer (NSCLC)." *JOURNAL OF CLINICAL ONCOLOGY*, Vol. 37, 26 May 2019 (2019-05-26), abstract | 1-19 |
| X | WATERSONE HANXBIO PTY LTD. "NCT04097769" *ClinicalTrials*, 23 September 2019 (2019-09-23), entire document | 1, 7-19 |
| Y | WATERSONE HANXBIO PTY LTD. "NCT04097769" *ClinicalTrials*, 23 September 2019 (2019-09-23), entire document | 1-19 |

Form PCT/ISA/210 (second sheet) (January 2015)

**INTERNATIONAL SEARCH REPORT**

International application No.

**PCT/CN2022/086890**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☐ forming part of the international application as filed:

        ☐ in the form of an Annex C/ST.25 text file.

        ☐ on paper or in the form of an image file.

    b. ☑ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
| --- |
| **PCT/CN2022/086890** |

| **Box No. II** | **Observations where certain claims were found unsearchable (Continuation of item 2 of first sheet)** |

This international search report has not been established in respect of certain claims under Article 17(2)(a) for the following reasons:

1. ☑      Claims Nos.: **16-17** （部分）
         because they relate to subject matter not required to be searched by this Authority, namely:

         [1]    Claims 16 and 17 (in part) relate to a method for treating or preventing a tumor, that is, claims 16
             and 17 (in part) relate to the subject matter as defined in PCT Rule 39.1(iv) that does not warrant a
             search conducted by the international searching authority: (4) a method for treating a human or animal
             body by surgery or therapy and a diagnostic method implemented on the human or animal body.
             An international search was formed on the basis of a use of effective amount of component A and
             component B in preparation of a drug for treating or preventing tumors.

2. ☐      Claims Nos.:
         because they relate to parts of the international application that do not comply with the prescribed requirements to such an
         extent that no meaningful international search can be carried out, specifically:

3. ☐      Claims Nos.:
         because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/086890**

| Patent document cited in search report | | | Publication date (day/month/year) | Patent family member(s) | | | Publication date (day/month/year) |
|---|---|---|---|---|---|---|---|
| CN | 112442123 | A | 05 March 2021 | None | | | |
| CN | 112300286 | A | 02 February 2021 | None | | | |
| CN | 112830972 | A | 25 May 2021 | WO | 2021104302 | A1 | 03 June 2021 |
| WO | 2021004480 | A1 | 14 January 2021 | CN | 114072426 | A | 18 February 2022 |
| | | | | EP | 3998287 | A1 | 18 May 2022 |

Form PCT/ISA/210 (patent family annex) (January 2015)

# EP 4 190 816 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 8801649 A
- US 4946778 A
- US 5260203 A
- US 4816567 A
- US 5747654 A

### Non-patent literature cited in the description

- **MATOZAKI T ; MURATA Y ; OKAZAWA H et al.** Functions and molecular mechanisms of the CD47-SIRPα signaling pathway. *Trends in cell biology,* 2009, vol. 19 (2), 72-80
- **OLDENBORG P A ; ZHELEZNYAK A ; FANG Y F et al.** Role of CD47 as a marker of self on red blood cells. *Science,* 2000, vol. 288 (5473), 2051-2054
- **JIANG P ; LAGENAUR CF ; NARAYANAN V.** Integrin-associated Protein Is a Ligand for the P84 Neural Adhesion Molecule. *Journal of Biological Chemistry,* 1999, vol. 274, 559-62
- **JAISWAL S ; JAMIESON C H M ; PANG W W et al.** *Cell,* 2009, vol. 138 (3), 271-285
- **VONDERHEIDE R H.** *Nature Medicine,* 2015, vol. 21 (10), 1122
- **WARD et al.** *Nature,* 1989, vol. 341, 544-546
- **LIU JIANJUN.** *Chinese Journal of Cellular and Molecular Immunology,* 1989, vol. 4, 29-29
- **BIRD et al.** *Science,* 1988, vol. 242, 423-426
- **HUSTON et al.** *Proc. Natl. Acad. Sci. USA.,* 1988, vol. 90, 5879-5883
- **SONGSIVILAI ; LACHMANN.** *Clin. Exp. Immunol.,* 1990, vol. 79, 315-321
- **KOSTELNY et al.** *J. Immunol.,* 1992, vol. 148, 1547-1553
- **KOHLER et al.** *Nature,* 1975, vol. 256, 495
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **CLARK.** *Immunol. Today,* 2000, vol. 21, 397-402
- Epitope Mapping Protocols in Methods in Molecular Biology. 1996, vol. 66
- **RAJAGOPAL et al.** *Prot. Engin.,* 1997, vol. 10, 1453-1459
- **REITER et al.** *Nat. Biotechnol.,* 1996, vol. 14, 1239-1245
- **REITER et al.** *Protein Engineering,* 1995, vol. 8, 1323-1331
- **WEBBER et al.** *Molecular Immunology,* 1995, vol. 32, 249-258
- **REITER et al.** *Immunity,* 1995, vol. 2, 281-287
- **REITER et al.** *JBC,* 1994, vol. 269, 18327-18331
- **REITER et al.** *Inter. J. of Cancer,* 1994, vol. 58, 142-149
- **REITER et al.** *Cancer Res.,* 1994, vol. 54, 2714-2718
- **PEARSON.** *Methods Mol. Biol.,* 1994, vol. 243, 307-31
- **GONNET et al.** *Science,* 1992, vol. 256, 1443-45
- **PEARSON.** *Methods Enzymol.,* 1990, vol. 183, 63-98
- **PEARSON.** *Methods Mol. Biol.,* 2000, vol. 132, 185-219
- **ALTSCHUL et al.** *Mol. Biol.,* 1990, vol. 215, 403-410
- **AL TSCHUL et al.** *Nucleic Acids Res.,* 1997, vol. 25, 3389-402
- Remington's Pharmaceutical Sciences. Mack Publishing Company, 1995
- **J. SAMBROOK et al.** Guide to Molecular Cloning Experiments. Science Press
- **ACIERNO et al.** Affinity maturation increases the stability and plasticity of the Fv domain of anti-protein antibodies. *J Mol biol.,* 2007, vol. 374 (1), 130-46
- WHO Drug Information, Proposed INN: List 124. *WHO Drug Information,* 2020, vol. 34 (4), 947-949
- WHO Drug Information, Proposed INN: List 123. *WHO Drug Information,* 2020, vol. 34 (2), 375-376
- **HAGE T ; REINEMER P ; SEBALD W.** Crystals of a 1:1 Complex Between Human Interleukin-4 and the Extracellular Domain of Its Receptor Alpha Chain. *Eur. J. Biochem.,* 1998, vol. 258 (2), 831-6

- Molecular Cloning: A Laboratory Manual